# EUROPEAN PATENT APPLICATION

(11) **EP 2 774 915 A1**
(43) Date of publication of application: **10.09.2014**
(21) Application number: 14157948.2
(22) Date of filing: 05.03.2014
(51) Int. Cl.: C07C 269/02, C07C 271/40, C07C 271/42

(54) **New derivatives of resveratrol**

(30) Priority: 06.03.2013 IT RM20130135
(71) Applicant: Mattarei, Andrea, 35131 Padova (IT); Biasutto, Lucia, 35030 Selvazzano Dentro PD (IT); Zoratti, Mario, 35127 Padova (IT); Paradisi, Cristina, 35127 Padova (IT); Marotta, Ester, 30035 Mirano VE (IT); Garbisa, Spiridione, 35037 Teolo (PD) (IT); Azzolini, Michele, 38068 Rovereto TN (IT); Bradaschia, Alice, 35142 Padova (IT); Carraro, Massimo, 30100 Campolongo Maggiore VE (IT); Sassi, Nicola, 24061 Albano Sant'Alessandro BG (IT)
(72) Inventor: Mattarei, Andrea, 35131 Padova (IT); Biasutto, Lucia, 35030 Selvazzano Dentro PD (IT); Zoratti, Mario, 35127 Padova (IT); Paradisi, Cristina, 35127 Padova (IT); Marotta, Ester, 30035 Mirano VE (IT); Garbisa, Spiridione, 35037 Teolo (PD) (IT); Azzolini, Michele, 38068 Rovereto TN (IT); Bradaschia, Alice, 35142 Padova (IT); Carraro, Massimo, 30100 Campolongo Maggiore VE (IT); Sassi, Nicola, 24061 Albano Sant'Alessandro BG (IT)
(74) Representative: Germinario, Claudio

(57) **Abstract**

A novel class of derivatives of resveratrol is provided which have favourable physico-chemical properties when compared with resveratrol itself. It is characterized by the presence of three carbamate functions, in which resveratrol is the oxygen-linked moiety, while the nitrogen atom carries a substituent conferring desirable characteristics to the molecule. These new compounds are especially suitable as precursors (prodrugs) and vehicles of resveratrol in preparations used as dietary integrators or in pharmaceutical compositions: they are stable towards oxidative degradation allowing for long shelf lifetimes and have suitable reactivity to regenerate and deliver resveratrol under physiological conditions.

## Description

A novel class of derivatives of resveratrol is provided which have favourable physico-chemical properties when compared with resveratrol itself. It is characterized by the presence of three carbamate functions, in which resveratrol is the oxygen-linked moiety, while the nitrogen atom carries a substituent conferring desirable characteristics to the molecule. These new molecules are especially suitable as precursors (prodrugs) and vehicles of resveratrol in preparations used as dietary integrators or in pharmaceutical compositions: they are stable towards oxidative degradation allowing for long shelf lifetimes and have suitable reactivity to regenerate and deliver resveratrol under physiological conditions.

### BACKGROUND ART

Resveratrol is a natural compound exhibiting biochemical activities of potential relevance for such major health-care endeavours as protecting the cardiovascular system, potentiating the immune response, contrasting inflammatory ailments such as arthritis, pathogenic infections, aging, obesity and metabolic disease, improving skeletal muscle functionality and intellectual performance impaired by age or neurodegeneration, and preventing or contrasting cancer (e.g.: Harikumar and Aggarwal, 2008; Parvaiz and Holme, 2009; Petrovski et al., 2011; Agarwal and Baur, 2011; Park et al., 2012). These activities account for the rapid growth of the market for resveratrol-based preparations and formulations used as dietary supplements, food supplements and self-prescription medicines. Two complications affecting this industry are the limited solubility of resveratrol in aqueous media and its susceptibility to oxidation. These characteristics restrict the modalities of administration, packaging and storage.

An approach to overcome these difficulties is offered by "prodrugs", i.e., derivatives of the compound of interest (in this case, resveratrol) which possess desirable characteristics and which can regenerate the parent active compound.

The patent literature contains claims to preparations based on resveratrol derivatives with part or all of the hydroxyls covalently engaged in functions such as alkoxy, phosphate, sulfonate, nitrate, carbonate, carboxyester (acyl) or glycoside (Pettit and Grealish, 2003; Soby et al., 2003; Maes et al., 2009; Lu et al., 2011). All these functions present shortcomings. The hydrolysis of some (carbonate, carboxyester) in aqueous solution or in a physiological environment is very facile, so that protection is too rapidly lost to be of any practical use. In other cases (alkyl ether, sulfonate, nitrate, the compounds described in Lu et al., 2011) hydrolysis is exceedingly slow or requires harsh conditions, preventing the regeneration of resveratrol on a useful timescale in a biologically relevant setting. Thus, these derivatives fall short of qualification as a prodrug. Other modifications reported may be suitable for the regeneration of the hydroxyl groups within the correct time window, but are limited in the scope of their applicability; phosphate and glycoside substituents, for example, will increase water solubility but are not suitable to modulate the properties of the molecule along the hydrophilicity-lipophilicity axis so as to optimize its properties for novel formulations. There is thus a need for a general method of protection with the versatility to be of general usefulness for applications in the nutritional and health fields. This new protection should be easy to implement, and advantageous also in terms of industrial and commercial aspects such as packaging and shelf life.

### SUMMARY OF THE INVENTION

The current state of the art leaves still unresolved, or only partially/unsatisfactorily resolved, the following major problems, addressed by this invention:
a) low aqueous solubility of resveratrol, with attendant constraints for its packaging and administration;
b) possible oxidative degradation of resveratrol in preparations;
c) unsatisfactory performance of available resveratrol prodrugs in terms of stability under physiological conditions;

The present invention solves these problems providing resveratrol prodrugs incorporating the carbamoyl group. Since the problems to be tackled are multi-faceted, the invention is multi-tasking, allowing its use under different circumstances and for different purposes depending on the N-linked substituent as part of the protecting group.

The invention is based on the serendipitous discovery of a protective functionality for the hydroxyls of resveratrol, the carbamate group, which conjugates near-optimal stability under physiologically relevant conditions with the versatility of conferring almost any desired physico-chemical characteristics to the prodrug.

The inventors found that the type of substituent group affects dramatically the properties of the resveratrol derivative, notably the hydro- and lipophilicity of the molecule. Prodrugs are presented which span the whole range of solubilities from hydrophilic, for use as aqueous solutions (point a), to lipophilic with good solubility in oils. In the resveratrol derivates of the present invention the carbamate group protects the hydroxyls against oxidation and the subsequent degradation of the molecule. The resveratrol derivatives of the invention are stable towards oxidation, in contrast to resveratrol itself (point b). Finally, the carbamate bond has good stability under acidic conditions, allowing for safe passage through the stomach as well as long-term (months) storage in a slightly acidic solution at refrigerator temperature without the need to add stabilizing agents. Yet, at physiological pH and in blood the N-monosubstituted carbamate bond is lysed with a near-ideal half-life time of - 50-200 minutes at 37 °C depending on the conditions - physiological pH or blood (point c). This invention furthermore represents an improvement in the synthetic procedures available for the synthesis of phenolic carbamates, as explained below in the description.

Accordingly, the present invention provides resveratrol derivatives having the formula (I): 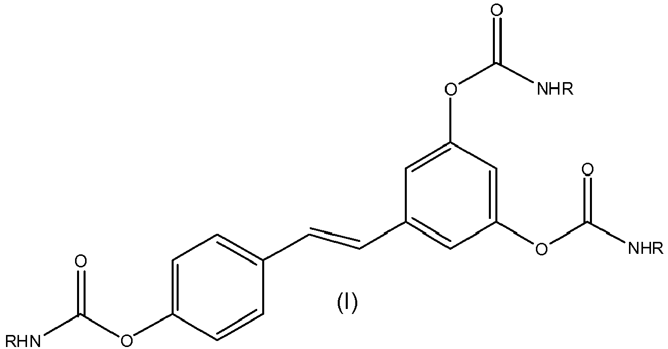 wherein each group NHR derives from the corresponding amine RNH₂ selected from the following compounds:
- methoxyoligoethylene glycol amines;
- methoxypolyethylene glycol amines with molecular mass lower than 20,000 g/mol;
- amino deoxy sugars;
- aliphatic amino alcohols;
- aliphatic amino polyalcohols with 1 to 10 carbon atoms;
- saturated or unsaturated aliphatic amines with 1 to 20 carbon atoms;
- natural or unnatural amino acids.

This invention also provides a pharmaceutical composition comprising a compound of formula (I) or a pharmaceutically acceptable salt, and a pharmaceutically acceptable carrier.

### BRIEF DESCRIPTION OF THE FIGURES

**Fig. 1****.** Chemical structure of the compounds deriving from this invention.
**Fig. 2****.** Time course of the concentrations of 3,4',5-tri-(N-(6-deoxy-galactosyl)-carbamoyl)-resveratrol (Resv(DGAL-C)₃) and its hydrolysis products in PBS at pH 6.8 (A) and in fresh rat blood (B).
**Fig. 3****.** Time course of the concentrations of 3,4',5-tri-(N-(2,3-hydroxypropyl)-carbamoyl)-resveratrol (Resv(MDHP-C)₃) and its hydrolysis products in PBS at pH 6.8 (A) and fresh rat blood (B).
**Fig. 4****.** Time course of the concentrations of 3,4',5-tri-(N-(6-deoxy-galactosyl)-carbamoyl)-resveratrol (50 µM) and of its hydrolysis products in 20 mM PBS, pH 5.0, at 4 °C (A) and at room temperature (B).
**Fig. 5****.** The reaction of DPPH with Resv(TGME-C)₃ and with resveratrol monitored by the decrease of DPPH absorbance at 520 nm. At time zero, 20 nmoles of DPPH and the specified amount of resveratrol or of its derivative were mixed in methanol. Absorbance was measured after 40 min.

### DETAILED DESCRIPTION OF THE INVENTION

The present invention provides compounds of resveratrol derivatives having the formula (I): 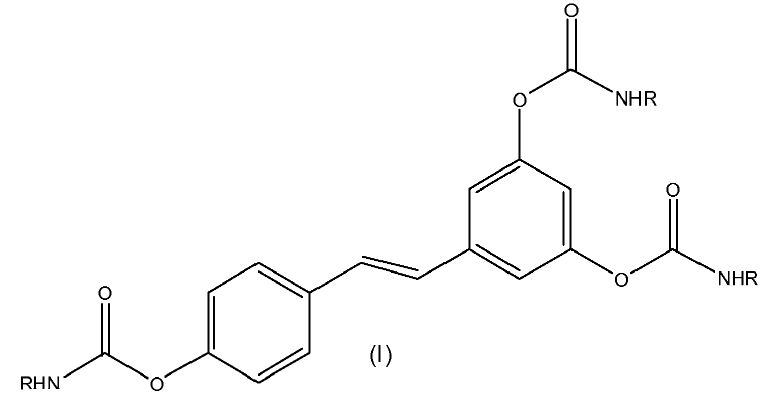 wherein each group NHR derives from the corresponding amine RNH₂ selected from the following compounds:
- methoxyoligoethylene glycol amines;
- methoxypolyethylene glycol amines with molecular mass lower than 20,000 g/mol;
- amino deoxy sugars;
- aliphatic amino alcohols;
- aliphatic amino polyalcohols with 1 to 10 carbon atoms;
- saturated or unsaturated aliphatic amines with 1 to 20 carbon atoms;
- natural or unnatural amino acids.

According to one embodiment of the invention the group -NHR derives from the corresponding methoxyoligoethylene glycol amine selected from the group consisting of methoxymonoethylene glycol amine, methoxydiethylene glycol amine, methoxytriethylene glycol amine, methoxytetraethylene glycol amine; methoxypentaethylene glycol amine, methoxyhexaethylene glycol amine, methoxyheptaethylene glycol amine, methoxyoctaethylene glycol amine, methoxynonaethylene glycol amine, methoxydecaethylene glycol amine, methoxypolyethylene glycol amine with an average Mₙ (molecular weight) of 2,000, methoxypolyethylene glycol amine with an average Mₙ of 5,000, methoxypolyethylene glycol amine with an average Mₙ of 10,000, methoxypolyethylene glycol amine with an average Mₙ of 20,000.

According to another embodiment of the invention the group -NHR derives from an amino deoxysugar selected from the following, including all possible isomers (regio-and stereoisomers): amino deoxy pentoses, amino deoxy hexoses, amino deoxy riboses, amino deoxy arabinoses, amino deoxy xyloses, amino deoxy lyxoses, amino deoxy alloses, amino deoxy altroses, amino deoxy glucoses, amino deoxy guloses, amino deoxy mannoses, amino deoxy galactoses, amino deoxy rhamnoses;

According to another embodiment of the invention the group -NHR derives from an aliphatic amino alcohol or polyalcohol selected from the following: aminomethanol, aminoethanol, aminoethandiol, aminopropanol, aminopropandiol, aminopropantriol, aminobutanol, aminobutandiol, aminobutantriol, aminobutantetraol, aminopentanol, aminopentandiol, aminopentantriol, aminopentantetraol, aminopentanpentaol, aminocyclopentanol, aminocyclopentandiol, aminocyclopentantriol, aminocyclopentantetraol, aminocyclopentanpentaol, aminohexanol, aminohexandiol, aminohexantriol, aminohexantetraol, aminohexanpentanol, aminohexanhexaol, aminocyclohexanol, aminocyclohexandiol, aminocyclohexantriol, aminocyclohexantetraol, aminocyclohexanpentaol, aminocyclohexanhexaol aminoheptanol, aminoheptandiol, aminoheptanetriol, aminoheptanetetraol, aminoheptanpentaol, aminoheptanhexaol, aminoheptanheptaol, aminooctanol, aminooctandiol, aminooctantriol, aminooctanetetraol, aminooctanpentaol, aminooctanhexaol, aminooctaneheptaol, aminooctaneoctaol, aminononanol, aminononandiol, aminononantriol, aminononantetraol, aminononanpentaol, aminononanhexaol, aminononanheptaol, aminononanoctaol, aminononannonaol, aminodecanol, aminodecandiol, aminodecantriol, aminodecantetraol, aminodecanpentaol, aminodecanhexaol, aminodecanheptaol, aminodecanoctaol, aminodecannonanol, aminodecandecanol, comprehensive of all their aliphatic amino alcohol or aliphatic amino polyalcohol isomers.

According to another embodiment of the invention the group -NHR derives from a saturated or unsaturated aliphatic amine selected from the following: methanamine, ethanamine, propanamine, butanamine, pentanamine, hexanamine, heptanamine, octanamine, nonanamine, decanamine, undecanamine, dodecanamine, tridecanamine, tetradecanamine, pentadecanamine, hexadecanamine, heptadecanamine, octadecanamine, nonadecanamine, icosanamine, tetradecenamine, hexadecenamine, heptadecenamine, octadecenamine, octadecadienamine, octadecatrienamine, icosatetraenamine, icosapentaenamine, comprehensive of all their saturated or unsaturated aliphatic amino hydrocarbon isomers.

According to another embodiment of the invention the group -NHR derives from one of the following natural or unnatural amino acids: alanine, arginine, asparagine, cysteine, aspartic acid, glutamic acid, glutamine, glycine, ornithine, proline, selenocysteine, serine, taurine, tyrosine, histidine, isoleucine, leucine, lysine, methionine, phenylalanine, threonine, tryptophan, valine, 2,4-diaminobutyric acid, 2-aminoheptanoic acid, 2-aminohexadecanoic acid, 5-aminovaleric acid, 4-hydroxy-proline, 3-phenylserine, α-methylvaline, 2-aminocaprylic acid, 2-amino-2-phenylbutyric acid, 2,6-diaminopimelic acid, comprehensive the D or L stereoisomer for each amino acid.

Preferred examples of compounds of the invention comprise:
- 3,4',5-tri-[N-(methoxy tri-(ethylenglycol))-carbamoyl] resveratrol;
- 3,4',5-tri-[N-(methoxy tetra-(ethylenglycol))-carbamoyl] resveratrol;
- 3,4',5-tri-[N-(methoxy hexa-(ethylenglycol))-carbamoyl] resveratrol;
- 3,4',5-tri-[N-(6-deoxy-galactosyl)-carbamoyl]-resveratrol;
- 3,4',5-tri-[N-(2,3-dihydroxypropyl)-carbamoyl] resveratrol;
- 3,4',5-tri-[N-methyl-carbamoyl] resveratrol;
- 3,4',5-tri-[N-(octadec-9-enyl)-carbamoyl]-resveratrol;
- 3,4',5-tri-[N-(glycyl)-carbamoyl]-resveratrol;
- 3,4',5-tri-[N-(leucyl)-carbamoyl]-resveratrol;
- 3,4',5-tri-[N-(isoleucyl)-carbamoyl]-resveratrol;
- 3,4',5-tri-[N-(threonyl)-carbamoyl]-resveratrol;
- 3,4',5-tri-[N-(phenylalanyl)-carbamoyl]-resveratrol.

Depending on the nature of substituents, R, two or more diastereoisomers may be possible. In that situation the present invention comprises the individual diastereoisomers and mixtures thereof.

Polymorphic forms of the compounds of formula (I) and of the salts, solvates and complexes are intended to be included in the present invention.

All stereoisomers (for example, geometric isomers, optical isomers and the like) of the present compounds (including those of the salts, solvates and complexes of the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate" "complex" and the like, is intended to equally apply to the salt, solvate and complex of enantiomers, stereoisomers, rotamers, tautomers, racemates or any other form of the inventive compounds.

Diasteromeric mixtures can be separated into their individual diastereomers on the basis of their physico-chemical differences by methods well known to those skilled in the art, such as, for example, by chromatography and/or fractional crystallization. Enantiomers can be separated by converting the enantiomeric mixture into a diasteromeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Enantiomers can also be separated by use of a chiral HPLC column. Also, some of the compounds of Formula (I) may be atropisomers (e.g., substituted biaryls) and are considered as part of this invention.

The compounds of formula (I) may form salts that are also within the scope of this invention. Reference to a compound of formula (I) herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of formula (I) contains both a basic moiety, such as, but not limited to an amino or imidazyl group, and an acidic moiety, such as, but not limited to, a carboxylic acid, zwitterions ("inner salts") may be formed and are included within the term "salt(s)" as used herein. Pharmaceutically acceptable (i.e., non-toxic, physiologically acceptable salts) are preferred. Salts of compounds of the formula (I) may be formed, for example, by reacting a compound of formula (I) with an amount of acid or base, e.g. an equivalent amount, in a medium such as one in which the salt precipitates or in an aqueous medium followed by lyophilization. Acids (and bases) which are generally considered suitable for the formation of pharmaceutically useful salts from basic (or acidic) pharmaceutical compounds are discussed, for example, by S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; in The Orange Book (Food & Drug Administration, Washington, D.C. on their website); and P. Heinrich Stahl, Camille G. Wermuth (Eds.), Handbook of Pharmaceutical Salts: Properties, Selection, and Use, (2002) Int'l. Union of Pure and Applied Chemistry, pp. 330-331. These disclosures are incorporated herein by reference thereto.

The compounds of this invention may be in crystalline or non-crystalline form, and, if crystalline, may optionally be solvated, especially hydrated. This invention includes within its scope stoichiometric hydrates as well as compounds containing variable amounts of water.

The compounds according to the invention may be suitably provided in substantially pure form, for example at least 50% pure, suitable at least 60% pure, advantageously at least 75% pure, preferably at least 85% pure, more preferably at least 95% pure, especially at least 98% pure, all percentages being calculated as weight/weight.

The compounds of the present invention and their pharmaceutically acceptable salts or derivatives have improved stability and solubility with respect to resveratrol and are therefore of use in therapy as medicaments in all cases and conditions for which resveratrol may exert beneficial effects.

In particular for treating inflammatory conditions, viral infections, bacterial infections of the upper respiratory tracts, metabolic disease, cancer, disorders of the gastro-intestinal tract, cardiovascular disease, traumatic brain injury in animals, especially mammals, including humans, in particular humans and domesticated animals (including farm animals).

The compounds may be used:
- for the treatment of viral infections and conditions, as for example the common cold, influenzas, herpes simplex, zoster, genitalis, bacterial infections of the upper respiratory tracts;
- for the treatment of metabolic disease as for example obesity, type II diabetes, management of cholesterol, triglyceride and sugar levels;
- as a co-adjuvant in the chemotherapy or other treatment of cancers;
- in inflammatory conditions such as rheumatoid arthritis, hay fever, rhinitis, inflamed/swollen nasal ducts, asthma, otitis, gingivitis and periodontitis;

The compounds may be used in the prevention and/or treatment of cardiovascular disease, including for example hardening of blood vessel walls, occlusion of blood vessels, infarct, ischemia/reperfusion injury; of aging-related dysfunctions - muscle weakness, loss of mental acuity, memory decline - and in the therapy of traumatic brain injury.

The compounds may be used for the treatment of disorders of skeletal muscle, of the central and peripheral nervous systems, of the endocrine, dermatological and genitourinary systems.

The compounds according to the invention may suitably be administered to the patient at a daily dosage for example from 0.05 to 50 mg/kg of body weight. Higher or lower dosages may, however, be used in accordance with normal clinical practice. Accordingly, in a further aspect, the present invention provides a composition comprising a compound of the invention or a pharmaceutically acceptable salt or derivative or solvate thereof together with a pharmaceutically acceptable carrier (or excipient).

Compositions according to the invention, in addition to the above, may also contain further compounds, for example: flavonoids, including in this term flavones (e.g. luteolin, apigenin), flavonols (e.g. quercetin, kaempferol), flavanones (e.g. naringenin), flavanolols (e.g. taxifolin), flavanols (e.g. catechin, catechin-3-gallate, epigallocateching-3-gallate), isoflavonoids (e.g. genistein, daidzein, equol), anthocyanidins (e.g. delphinidin, malvidin); curcumin; organosulphur (e.g. sulphoraphane, N-acetylcysteine) and organoselenium (e.g. selenocysteine) compounds; vitamins (e.g. vitamin A, carotenoids, vitamins of the B complex (e.g. biotin, riboflavin, niacin, folic acid), vitamin C, vitamin E (tocopherols)); omega-3-fatty acids and their esters; Coenzyme Q₁₀; carnitine; aminoacids; peptides; cyclodextrins. These further compounds may be present as natural derivatives (e.g. glycosides), prodrugs (e.g. esters), oligomers or polymers, salts. They may furthermore be present as components of extracts or preparations obtained from herbs or plants. The composition may also contain inorganic salts and ions, such as those of Mg, Zn, Ca, and their complexes.

The compositions of the present invention may be used in the treatment of the diseases described above and may be formulated for administration by any route, for example oral, topical or parenteral. The compositions may, for example, be made up in the form of tablets, capsules, powders, granules, lozenges, creams, syrups, sprays or liquid preparations, for example solutions or suspensions, which may be formulated for oral use or in sterile form for parenteral administration by injection or infusion.

More generally, the compounds and compositions according to the invention may be formulated for administration in any convenient way for use in human or veterinary medicine.

Tablets and capsules for oral administration may be in unit dosage form, and may contain conventional excipients including, for example, binding agents, for example, syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or another suitable vehicle before use. Such liquid preparations may contain conventional additives, including, for example, suspending agents, for example sorbitol, methyl cellulose, glucose syrup, gelatin, hydroxyethyl cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and colouring agents.

Compositions according to the invention intended for topical administration may, for example, be in the form of ointments, creams, lotions, eye ointments, eye drops, ear drops, nose drops, nasal sprays, impregnated dressings, and aerosols, and may contain appropriate conventional additives, including, for example, preservatives, solvents to assist drug penetration, and emollients in ointments and creams. Such topical formulations may also contain compatible conventional carriers, for example cream or ointment bases, ethanol or oleyl alcohol for lotions and aqueous bases for sprays. Such carriers may constitute from about 1% to about 98% by weight of the formulation; more usually they will constitute up to about 80% by weight of the formulation.

Compositions according to the invention intended for topical administration, in addition to the above, may also contain further compounds, for example: viniferins, flavonoids, coenzyme Q₁₋₁₀, vitamins (e.g. vitamin C, vitamin A), xanthophyillins, hyaluronic acid. These compounds may be present as derivatives or prodrugs (e.g. esters), oligomers or polymers, salts. The composition may also contain inorganic salts and ions, such as those of Mg, Zn, Ca, and their complexes.

Compositions according to the invention intended for parenteral administration may conveniently be in fluid unit dosage forms, which may be prepared utilizing the compound and a sterile vehicle, water being preferred. The compound, depending on the vehicle and concentration used, may be either suspended or dissolved in the vehicle. In preparing solutions, the compound may be dissolved in water for injection and filter-sterilised before being filled into a suitable vial or ampoule, which is then sealed. Advantageously, conventional additives including, for example, local anaesthetics, preservatives, and buffering agents can be dissolved in the vehicle. In order to enhance the stability of the solution, the composition may be frozen after being filled into the vial, and the water removed under vacuum; the resulting dry lyophilised powder may then be sealed in the vial and an accompanying vial of water for injection may be supplied to reconstitute the liquid prior to use. Parenteral suspensions may be prepared in substantially the same manner except that the compound is suspended in the vehicle instead of being dissolved and sterilisation cannot be accomplished by filtration. The compound may instead be sterilised by exposure to ethylene oxide before being suspended in the sterile vehicle. Advantageously, a surfactant or wetting agent may be included in such suspensions in order to facilitate uniform distribution of the compound.

A composition according to the invention may suitably contain for example from 0.03 to 95 % by weight of a compound of the invention or of a salt, derivative or solvated form thereof. When the compositions according to the invention are presented in unit dosage form, for instance as a tablet, each unit dose may suitably comprise for example from 3 to 1000 mg of a compound described herein.

It is also an object of the present invention a cosmetic composition comprising a compound herein described and a suitable carrier for topic administration. The composition may contain other ingredients, e.g. retinol, alpha-hydroxyacids (e.g. glycolyc acid, lactic acid), omega-3 fatty acids, coenzyme Q₁₋₁₀, vitamins (e.g. vitamin C, vitamin A), sunscreen ingredients (e.g. titanium oxide, avobenzone, photostabilizers), cyclodextrins, detergents (e.g. laurylsulphate, cationic detergents) collagen derived peptides and hyaluronic acid. The carrier may be in the form of a liquid, oil, unguent, cream, paste, gel, suspension, lotion, powder, and may itself be a formulation of ingredients such as, e.g., glycerine, vaseline, lanolin, castor oil and derivatives, beeswax, talcum, corn starch, cellulose and its derivatives.

In particular embodiments, the composition or mixture comprising the compounds of the invention may be realized in the form of food supplement, food, nutraceutical or medical device by adding for example one or more excipients and/or food ingredients. There are no particular limitations in the realization as a supplement, food, nutraceutical or medical device, according to the present description. The compounds of the invention are suitable in all forms, commonly used by the technician in the sector, whose preparation does not eliminate the activity of active ingredients used. As a supplement the mixture or composition may be made in solid, semi-solid, gelatinous or liquid form, such as for example in the form of bars, candies, jellies, beverages (suspensions or emulsions) or in the form of food and more complex formulations, the preparation of which does not deteriorate the active ingredients of said composition. A non-limiting set of examples of such nutraceuticals may be represented, for example, by liquid beverages such as milkshakes, juices, soft drinks or foods such as candy, cookies, powders, granules, bars. The composition may also be introduced at the most appropriate time of the preparation, in liquid form or dried, in liquid or solid foods, as the technician in the sector of the production of food supplements may define in a simple way and without use of inventive step. The dietary supplement or food may also contain other ingredients including components such as combinations of vitamins, minerals and other substances aimed at the integration of the diet.

The synthetic procedures used to obtain the above-mentioned derivatives of resveratrol are illustrated in general terms below, while detailed examples are provided later.

### Methods for the synthesis of N-monosubstituted carbamate ester resveratrol derivatives

A further object of the present invention is a process for synthesizing the compounds described herein according to the following scheme 1; R is as defined in connection with the definition of the substituents of compounds of formula (I).

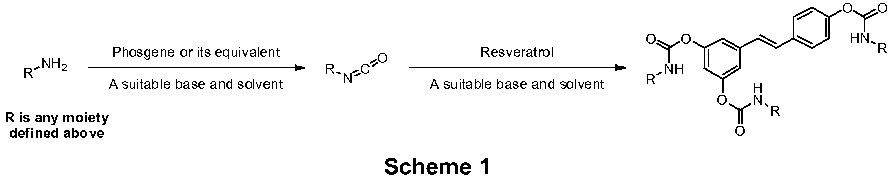

As shown in scheme 1, the N-monosubstituted carbamate ester linkage can be formed in two steps by reaction of the desired primary amine with phosgene or its equivalents such as diphosgene or triphosgene to give a reactive isocyanate derivative (which may also be commercially available), which is then coupled with the phenolic functions of resveratrol in the presence of a suitable base and solvent. Bases such as triethylamine, diisopropylethylamine, 4-(dimethylamino)pyridine, pyridine, sodium hydride and the like can be used. Suitable solvents include dichloromethane (CH₂Cl₂), chloroform (CHCl₃), N,N-dimethylformamide (DMF), tetrahydrofuran (THF), acetonitrile (ACN), ethyl acetate (EtOAc).

The invention also provides a novel improved method for the synthesis of the above-mentioned N-monosubstituted carbamate ester linkage, as shown in scheme 2.

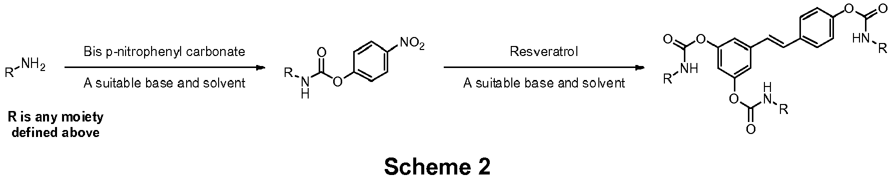

This new procedure involves reaction of the desired primary amine with bis-p-nitrophenyl carbonate to produce the p-nitrophenol carbamate ester intermediate which is easily converted into the desired product by reaction with resveratrol. This method provides a significantly improved synthetic approach for the production of phenolic carbamates in general as well as of resveratrol carbamates in the present invention. R is as defined in connection with the definition of the substituents of compounds of formula (I).

Suitable bases and solvents are the same as defined above.

Protection/deprotection steps, not shown here, may also be necessary if the amine contains potentially interfering reactive groups, as exemplified in sect. VIII and known by the person skilled in the art. The primary amines used as one of the starting materials can be conveniently produced via reduction of the corresponding azide with triphenylphosphine (Staudinger reaction). Azides can be easily produced from halides or tosylates by sodium azide substitution with excellent yields.

The following Examples illustrate the present invention and particularly the preparative procedures outlined above, by reference to the preparation of specific compounds within the scope of the present invention.

### Examples

### ANALYTICAL METHODS

The formation of the desired products, their purities and identities can be verified by means of the usual analytical methods employed in organic synthesis (HPLC, LC/MS, NMR). In particular, for HPLC analysis the gradient protocol has been optimized case by case, depending on the hydrophilicity/hydrophobicity of the derivatives to be analyzed. For example, in the analysis of hydrophilic compounds (e.g., those containing sugar moieties) we have used a rapid resolution (RRLC) - reverse phase column (Zorbax Eclipse Plus C18, 1.8 µm, 2.1 x 50 mm i.d.; Agilent); solvents A and B were in most cases H₂O containing 0.1% TFA and CH₃CN, respectively, and a typical gradient for B was as follows: 2% for 0.5 min, from 2% to 10% in 0.3 min, from 10% to 18% in 1.7 min, from 18% to 28% in 0.5 min, from 28% to 100% in 1.8 min (0.9 min), with a flow rate of 0.6 mL/min, injection volume 2 µl. The eluate was preferentially monitored at 286 nm for 4,4'-dihydroxybiphenyl (internal standard), 320 nm for resveratrol and its derivatives.

The stability of the prodrugs with respect to hydrolysis and the regeneration of resveratrol can be assessed by a) incubation in solutions of appropriate composition and pH and b) incubation in blood followed by separation and recovery of the small-molecule fraction and analysis. The susceptibility to oxidation can be assessed by studying c) the reaction with the 1,1-diphenyl-2-picrylhydrazyl (DPPH) radical. Experimental details are provided.

### METHODS FOR PRODRUG CHARACTERIZATION

*a) Stability in solution.* To determine the kinetics of hydrolysis the compound was dissolved (5 µM) in 0.1 N HCl or in PBS (pH 6.8). The solutions were incubated at 37 °C. Samples were taken and analysed directly by HPLC at the times indicated in the figures.
*b) Stability*/*metabolism in blood.* For kinetics in blood, the compound was added to fresh heparinized and EDTA-supplemented blood and at the desired times recovery work-up was carried out essentially as described in Biasutto et al. (2010).
c) *Stability to oxidation.* DPPH is a stable free radical with an absorbance maximum at 520 nm (purple). Upon reaction with polyphenols it is converted to DPPHH (yellow) and this change of colour is an indication of the extent of the reaction which has taken place. The assays were conducted using 96-well plates following Fukumoto and Mazza (2000), measuring absorbance with a Tecan Infinite 200 plate reader.
   *Solubility in aqueous media.* A calibration curve is constructed via HPLC analysis of a series of solutions of the compound of interest at known concentrations in a suitable solvent. A known amount (excess) of solid compound is placed in a 20-mL vial to which a 5 mL aliquot of 1 mM acetate buffer at pH 5 is then added. The contents are stirred at 25°C for 24 hours. After a 4-hour decantation period, 1 mL of the supernatant is filtered through a PTFE 0.45 µm filter, diluted with 2 v/v of acetonitrile and analyzed by HPLC. The entire procedure is repeated twice with the same sample, at 24-hour intervals, to verify the invariance of the result.
   *Octanol*/*water partition coefficient (LogP).* This parameter was estimated using the ALOGPS 2.1 software (Tetko and Bruneau, 2004), available online at http://www.vcclab.org/lab/alogps/start.html)

### Example 1 Synthesis of 3,4',5-tri-[N-(methoxy tri-(ethylenglycol))-carbamoyl] resveratrol (Resv(TGME-C)₃)

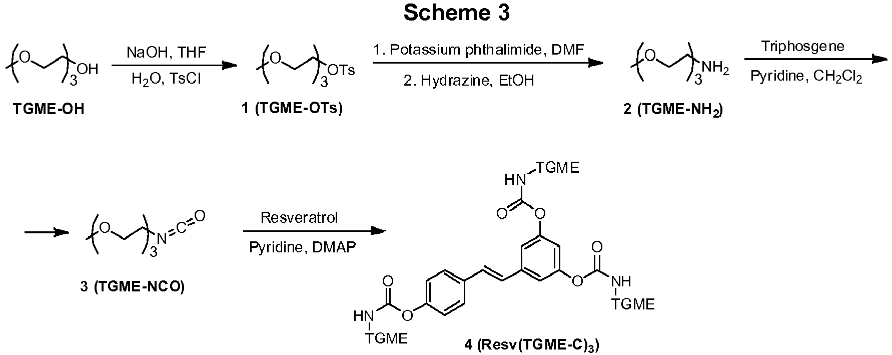

**Methoxy tri-(ethyleneglycol)-(p-toluensulfonate) (1; TGME-OTs):** Sodium hydroxide (2.4 g, 61.0 mmol, 2 eq.) in water (25 mL) and methoxy-tri-(ethylene glycol) (TGME-OH) (5.0 g, 31 mmol, 1 eq.) in THF (25 mL) were cooled in an ice-water bath with stirring and p-toluene sulfonyl chloride (11.6 g, 61.0 mmol) in THF (25 mL) was added dropwise over 30 min. The reaction mixture was stirred for an additional 2 h at 5 °C, poured into ice-water (100 mL) and extracted with dichloromethane (5 × 50 mL). The combined organic extracts were washed with water (2 × 50 mL) and saturated sodium chloride (1 × 50 mL), dried with MgSO₄ and the solvent removed *in vacuo.* The residue was purified by flash chromatography using CH₂Cl₂:EtOAc 8:2 as eluent to afford 7.2 g of 1 (74 %) as a colourless oil. ¹H-NMR (250 MHz, CDCl₃) δ (ppm): 2.43 (s, 3H, ArC*H₃*), 3.35 (s, 3H, -O-C*H₃*), 3.49-3.66 (m, 10H, 2 × -O-C*H₂*-C*H₂*-O- + -O-C*H₂*-), 4.14 (t, 2H, Ts-C*H₂*-, ³J_{H-H} = 5.75 Hz), 7.32 (d, 2H, 2 × Ar-H, ³J_{H-H} = 8.25 Hz), 7.77 (d, 2H, 2 × Ar-H, ³J_{H-H} = 8.25 Hz); ¹³C-NMR (250 MHz, CDCl₃) δ (ppm): 144.7, 132.9, 129.7, 127.9, 71.8, 70.6, 70.5, 70.4, 69.2, 68.6, 58.9, 21.6, 144.7, 132.9, 129.7, 127.9, 71.8, 70.6, 70.5, 70.4, 69.2, 68.6, 58.9, 21.6; ESI-MS (ion trap): m/z 337 [M+H₂O+H]⁺.

**Methoxy tri-(ethyleneglycol)-amine (2; TGME-NH₂): 1** (4.7 g, 15 mmol, 1 eq.) and potassium phtalimide (27.8 g, 150 mmol, 10 eq.) were dissolved in DMF (100 mL), and heated at 120 °C for 4 hours. Diethyl ether (300 mL) was then added, solids were removed by filtration and the precipitate was washed with diethyl ether (2 × 50 mL) and cold CH₂Cl₂ (2 × 50 mL). The filtrate was dried in vacuum and dissolved in 400 mL of ethanol. Hydrazine hydrate (7.5 g, 150 mmol, 10 eq.) was added and the resulting mixture was heated under reflux for 6 hours. The resulting mixture was then acidified to pH 1 with HCl (37%), filtered and concentrated. NaOH (50 mL, 30% in water) was then added and the resulting mixture was extracted with chloroform (5 × 70 mL). The combined extracts were dried in vacuum. The residue was diluted with water (100 mL), washed with diethyl ether (3 × 30 mL) and dried in vacuum to afford 2.1 g of **2** (86%) as pale yellow oil. ¹H-NMR (250 MHz, CDCl₃) δ (ppm): 1.57 (s, 2H, -N*H₂*), 2.79 (t, 2H, - C*H₂*-N*H₂*, ³J_{H-H} = 5.25 Hz) 3.31 (s, 3H, -O-C*H₃*), 3.42-3.60 (m, 10H, 2 × -O-C*H₂*-C*H₂*-O-*+ -*O-C*H₂-*); ¹³C-NMR (250 MHz, CDCl₃) δ (ppm): 73.2, 71.6, 70.3, 70.3, 70.0, 58.8, 41.5; ESI-MS (ion trap): m/z 164 [M+H]⁺.

**3,4',5-tri-[N-methoxy tri-(ethyleneglycol)-carbamoyl] resveratrol (4; Resv(TGME-C)₃):** Pyridine (3 mL) was added to a solution of triphosgene (1.92 g, 6.5 mmol, 1 eq.) in CH₂Cl₂ (20 mL), and the mixture was stirred at 0 °C for 20 min. A solution of **2** (1.05 g, 6.5 mmol, 1 eq.) in CH₂Cl₂ (20 mL) was then added dropwise and the mixture was stirred at room temperature for 45 minutes. After adding H₂O (50 mL), the mixture was extracted with CHCl₃ (3 × 50 mL). The combined organic layers were dried over MgSO₄ and filtered. The solvent was evaporated under reduced pressure to give the TGME-isocyanate **(3)** with traces of pyridine, which was used without further purification. A solution of resveratrol (0.28 g, 1.2 mmol, 0.2 eq.) and DMAP (0.59 g, 4.8 mmol, 0.75 eq.) in pyridine (5 mL) was added dropwise to the above solution and the resulting mixture was allowed to react under vigorous stirring at 50 °C overnight. The reaction mixture was then diluted in CH₂Cl₂ (100 mL) and washed with 0.5 N HCl (7 × 100 mL). The organic layer was dried over MgSO₄ and filtered. The solvent was evaporated under reduced pressure and the residue was purified by flash chromatography using CH₂Cl₂:acetone 6.5:3.5 as eluent to afford 0.53 g of **4** (55 %) as a yellow oil. ¹H-NMR (250 MHz, CDCl₃) δ (ppm): 3.08 (m, 9H, 3 × N-C*H₃*), 3.39 (s, 9H, 3 × -O-C*H₃*) 3.42-3.69 (m, 36H, 6 × -O-C*H₂*-C*H₂*-O- + 3 × -O-C*H₂*- + 3 × -N-C*H₂*-), 5.80 (m, 3H, 3 × -N*H*-), 6.86 (t, 1 H, ⁴J_{H,H} = 2.0 Hz, H-4), 6.90-7.12 (m, 6H, H-2, H-3', H-5', H-6, H-7, H-8), 7.45 (d, 2H, ³J_{H,H} = 8.75 Hz, H-2', H-6'); ¹³C-NMR (250 MHz, CDCl₃) δ (ppm): 154.6, 154.3, 151.6, 150.7, 139.1, 133.9, 129.2, 127.4, 127.1, 121.8, 116.3, 114.3, 71.8, 70.5, 70.2, 70.2, 69.8, 59.0, 40.9; ESI-MS (ion trap): m/z 818 [M+Na]⁺.
**Solubility in water:** > 10⁻² M
**Log P:** 2.36

### Example 2 Synthesis of 3,4',5-tri-[N-(methoxy hexa-(ethylenglycol))-carbamoyl] resveratrol (Resv(HexaGME-C)₃)

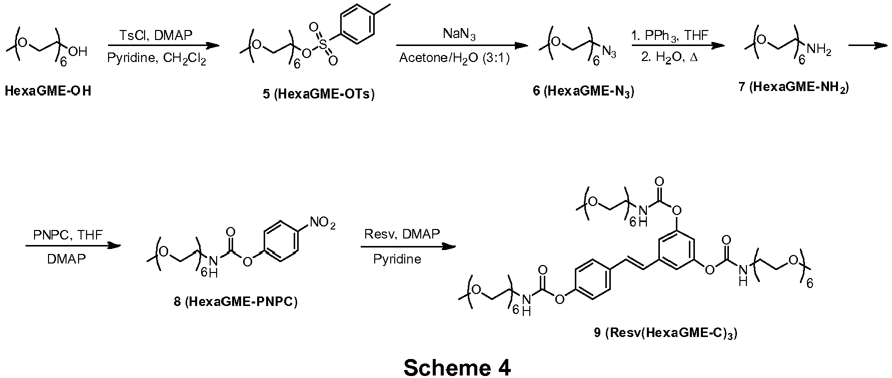

**Methoxy hexa-(ethyleneglycol)-(p-toluensulfonate) (5; HexaGME-OTs):** Pyridine (1.09 mL, 13.5 mmol, 2 eq.) and DMAP (1.65 g, 13.5 mmol, 2 eq.) were added to a solution of methoxy-hexa-(ethylene glycol) (HexaGME-OH) (2.0 g, 6.75 mmol, 1 eq.) in CH₂Cl₂ (10 mL), and the mixture was stirred at 0 °C for 15 min. A solution of tosyl chloride (1.93 g, 10.1 mmol, 1.5 eq.) in CH₂Cl₂ (10 mL) was then added dropwise and the mixture was stirred at room temperature for 4 hours. The mixture was diluted in CH₂Cl₂ (150 mL) and washed with 0.5 N HCl (100 mL). The aqueous layer was washed with CH₂Cl₂ (5 × 75 mL) and all the organic fractions were collected, dried over MgSO₄ and filtered. The solvent was evaporated under reduced pressure and the residue was purified by flash chromatography using CH₂Cl₂:acetone 65:35 as eluent to afford 3.0 g of **5** (98 %) as a colourless oil. ¹H-NMR (250 MHz, CDCl₃) δ (ppm): 2.35 (s, 3H, Ar-C*H₃*), 3.27 (s, 3H, -O-C*H₃*), 3.42-3.60 (m, 22H, 2 × -O-C*H₂*-C*H₂*-O- + -O-C*H₂*-), 4.05 (t, 2H, Ts-C*H₂*-, ³J_{H-H} = 5.00 Hz), 7.25 (d, 2H, 2 × Ar-*H*, ³J_{H-H} = 7.93 Hz), 7.70 (d, 2H, 2 × Ar-*H*, ³J_{H-H} = 8.34 Hz); ¹³C-NMR (62.9 MHz, CDCl₃) δ (ppm): 144.6, 132.7, 129.6, 127.7, 71.7, 70.5, 70.4, 70.4, 70.3, 70.3, 70.3, 69.1, 68.4, 58.8, 21.4; ESI-MS (ion trap): m/z 451 [M+H]⁺.

**Methoxy hexa-(ethyleneglycol)-(azide) (6; HexaGME-N₃):** Sodium azide (10.72 g, 0.165 mol, 5 eq.) was added to a solution of **5** (14.9 g, 33 mmol, 1 eq.) in a water:acetone solution (1:3, 65 mL), and the mixture was stirred at 75 °C overnight. The mixture was then diluted in CH₂Cl₂ (250 mL) and washed with water (250 mL). The aqueous layer was washed with CH₂Cl₂ (5 × 100 mL) and all the organic fractions were collected, dried over MgSO₄ and filtered. The solvent was evaporated under reduced pressure and the residue was purified by flash chromatography using CH₂Cl₂:acetone 8:2 as eluent to afford 10.6 g of **6** (100 %) as a colourless oil. ¹H-NMR (250 MHz, CDCl₃) δ (ppm): 3.28-3.32 (m, 5H, -O-C*H₃* + -O-CH₂-C*H₂*-N₃), 3.43-3.48 (m, 2H, -O-C*H₂*-CH₂-N₃), 3.53-3.61 (m, 20H, 5 × -O-C*H₂*-C*H₂*-O-); ¹³C-NMR (62.9 MHz, CDCl₃) δ (ppm): 71.6, 70.4, 70.4, 70.3, 70.3, 70.3, 70.3, 70.3, 70.2, 69.7, 58.7, 50.4; ESI-MS (ion trap): m/z 322 [M+H]⁺.

**Methoxy hexa-(ethyleneglycol)-(amine) (7; HexaGME-NH₂):** Triphenylphosphine (10.88 g, 41.5 mmol, 1.25 eq.) in 25 mL of anhydrous THF was added dropwise to a solution of methoxy-hexa-(ethylene glycol)-azide **6** (10.6 g, 33 mmol, 1 eq.) in anhydrous THF (25 mL), and the solution was stirred at RT for 5 hours. Distilled water (20 mL) was then added and the mixture was heated under reflux (80°C) and vigorously stirred overnight. The resulting mixture was evaporated under reduced pressure and the residue was purified by flash chromatography using CH₂Cl₂:MeOH 9:1 (+ 1% Et₃N) as eluent to afford 9.55 g of 7 (98 %) as a pale yellow oil. ¹H-NMR (250 MHz, CDCl₃) δ (ppm): 1.78 (s, 2H, -CH₂-N*H₂*), 2.76 (t, 2H, ³J_{H-H} = 5.25 Hz, -CH₂-C*H₂*-NH₂), 3.26 (s, 3H, -O-C*H₃*), 3.39-3.46 (m, 4H, -O-CH₂-C*H₂*-O- + -O-C*H₂*-CH₂-NH₂), 3.51-3.56 (m, 18H, 4 × -O-C*H₂*-C*H₂*-O- + -O-C*H₂*-CH₂-O-); ¹³C-NMR (62.9 MHz, CDCl₃) δ (ppm): 72.8, 71.6, 70.3, 70.3, 70.3, 70.2, 70.2, 70.2, 70.0, 58.7, 41.4; ESI-MS (ion trap): m/z 296 [M+H]⁺.

**[Methoxy hexa-(ethyleneglycol)-carbamoyl]-p nitrophenol (8; HexaGME-PNPC):** Bis(4-nitrophenyl) carbonate (2.74 g, 9.0 mmol, 1.1 eq.) in 15 mL of acetonitrile was added dropwise to a solution of methoxy-hexa-(ethylene glycol)-amine **7** (2.4 g, 8.2 mmol, 1 eq.) and DMAP (2.00 g, 16.4 mmol, 2 eq.) in 15 mL of acetonitrile and the resulting mixture was stirred at 50 °C for 3 hours. The mixture was diluted in CH₂Cl₂ (150 mL) and washed with 0.5 N HCl (100 mL). The aqueous layer was washed with CH₂Cl₂ (5 × 100 mL) and all the organic fractions were collected, dried over MgSO₄ and filtered. The solvent was evaporated under reduced pressure and the residue was purified by flash chromatography using CH₂Cl₂:acetone from 8:2 to 6:4 as eluent to afford 3.2 g of **9** (84 %) as a colourless oil. ¹H-NMR (250 MHz, CDCl₃) δ (ppm): 3.30 (s, 3H, -O-C*H₃*), 3.37-3.50 (m, 4H, -O-C*H₂*-C*H₂*-NH-), 3.55-3.61 (m, 20H, 5 × -O-C*H₂*-C*H₂*-O-), 6.04 (t, 1H, ³J_{H-H} = 5 Hz -CH₂-N*H*-CO-O-), 7.26 (d, 2H, ³J_{H-H} = 9.25 Hz, Ar-H), 8.17 (d, 2H, ³J_{H-H} = 9.25 Hz, Ar-H); ¹³C-NMR (62.9 MHz, CDCl₃) δ (ppm): 125.8, 124.9, 121.8, 115.5, 71.6, 70.3, 70.3, 70.3, 70.2, 70.0, 69.3, 58.8, 40.9; ESI-MS (ion trap): m/z 461 [M+H]⁺.

**3,4',5-tri-[N-methoxy hexa-(ethyleneglycol)-carbamoyl] resveratrol (9; Resv(HexaGME-C)₃):** 8 (0.13 g, 2.8 mmol, 5 eq.) in pyridine (5 mL) was added to a solution of resveratrol (0.13 g, 0.56 mmol, 1 eq.) and DMAP (0.27 g, 2.2 mmol, 2 eq.) in pyridine (5 mL), and the mixture was stirred at 50 °C overnight. The resulting mixture was then diluted in CH₂Cl₂ (150 mL) and washed with 1 N HCl (200 mL). The aqueous layer was washed with CH₂Cl₂ (5 × 100 mL) and all the organic fractions were collected, dried over MgSO₄ and filtered. The solvent was evaporated under reduced pressure and the residue was purified by flash chromatography using CH₂Cl₂:acetone from 4:6 to 2:8 as eluent to afford 0.51 g of **9** (76 %) as a colorless oil. 1 H-NMR (250 MHz, CDCl₃) δ (ppm): 3.33 (s, 9H, 3 × -O-CH3), 3.38-3.51 (m, 12H, 3 × -O-CH₂-CH₂-NH-), 3.57-3.68 (m, 60H, 15 × -O-CH₂-CH₂-O-), 5.80-5.86 (m, 3H, 3 × -CH₂-NH-CO-O-), 6.80-7.12 (m, 7H, H-2, H-4, H-3', H-5', H-6, H-7, H-8), 7.41 (d, 2H, 3JH,H = 8.75 Hz, H-2', H-6'); 13C-NMR (62.9 MHz, CDCl₃) δ (ppm): 154.4, 154.1, 151.5, 150.6, 138.9, 133.8, 129.1, 127.3, 127.0, 121.7, 116.2, 114.2, 71.7, 70.4, 70.4, 70.3, 70.3, 70.3, 70.2, 69.7, 58.8, 40.9; ESI-MS (ion trap): m/z 1193 [M+H]⁺.
**Solubility in water:** > 2*10⁻¹ M
**Log P:** 0.18

### Example 3 Synthesis of 3,4',5-tri-[N-(6-deoxy-galactosyl)-carbamoyl]-resveratrol (Resv(DGAL-C)₃)

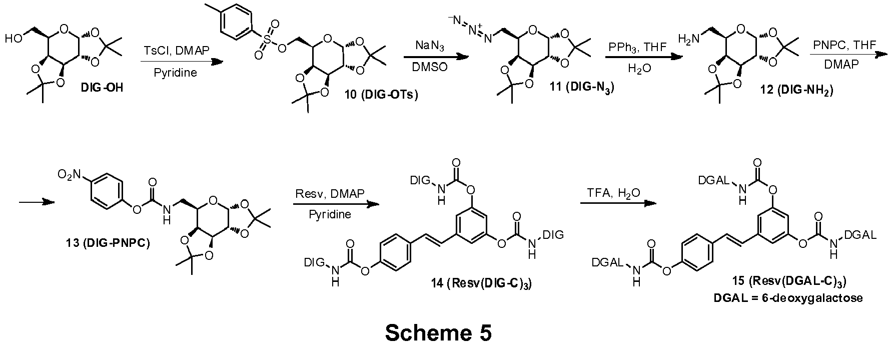

**(1,2:3,4-di-*O*-isopropylidene-6-*O*-(p-toluensulfonate))-D-galactopiranose (10; DIG-OTs):** Pyridine (3.1 mL, 38.4 mmol, 2 eq.) and DMAP (3.51 g, 28.7 mmol, 1.5 eq.) were added to a solution of 1,2:3,4-di-*O*-isopropylidene-D-galactopiranose (DIG-OH) (5.0 g, 19.2 mmol, 1 eq.) in CH₂Cl₂ (20 mL), and the mixture was stirred at 0 °C for 15 min. A solution of tosyl chloride (5.49 g, 28.7 mmol, 1.5 Eq) in CH₂Cl₂ (20 mL) was then added dropwise and the mixture was stirred at room temperature for 5 hours. After adding H₂O (30 mL), the mixture was diluted in CH₂Cl₂ (150 mL) and washed with 0.5 N HCl (5 × 100 mL). The organic layer was dried over MgSO₄ and filtered. The solvent was evaporated under reduced pressure and the residue was purified by flash chromatography using CH₂Cl₂:EtOAc 9:1 as eluent to afford 7.33 g of **10** (92 % yield). ¹H-NMR (250 MHz, CDCl₃) δ (ppm): 1.26 (s, 3H, -C-C*H₃*), 1.30 (s, 3H, -C-C*H₃*), 1.33 (s, 3H, -C-C*H₃*), 1.49 (s, 3H, -C-C*H₃*), 2.43 (s, 3H, Ar-C*H₃*), 4.00-4.20 (m, 4H, H-4, H-5, H-6), 4.28 (dd, 1 H, ³J₃₋₂ = 2.5 Hz, ³J₃₋₄ = 2.5 Hz, H-3), 4.57 (dd, 1 H, ³J₂₋₁ = 2.5 Hz, H-2), 5.44 (d, 1 H, ³J₁₋₂ = 5 Hz, H-1), 7.32 (d, 2H, Ar-*H*, ³J_{H-H} = 10 Hz), 7.80 (d, 2H, ³J_{H-H} = 7.5 Hz); ¹³C-NMR (250 MHz, CDCl₃) δ (ppm): 144.7, 132.7, 129.7, 128.0, 109.5, 108.9, 96.0, 70.4, 70.3, 70.3, 68.1, 65.8, 25.9, 25.7, 24.9, 24.3, 21.6; ESI-MS (ion trap): m/z 415 [M+H]⁺.

**(1,2:3,4-di-*O*-isopropylidene-6-deoxy-azido)-D-galactopiranose (11; DIG-N₃):** Sodium azide (5.84 g, 89.8 mmol, 5 eq.) was added to a solution of **10** (7.22 g, 17.4 mmol, 1 eq.) in DMSO (40 mL), and the mixture was stirred at 160 °C for 1.5 hours under nitrogen. The resulting mixture was poured into ice water (500 mL), and extracted with EtOAc (2 × 250 mL). The organic layer was dried over MgSO₄ and filtered. The solvent was evaporated under reduced pressure and the residue was purified by flash chromatography using CH₂Cl₂:EtOAc 97:3 as eluent to afford 4.60 g of **11** (93 % yield). ¹H-NMR (250 MHz, CDCl₃) δ (ppm): 1.33 (s, 6H, 2 × -C-C*H₃*), 1.44 (s, 3H, -C-C*H₃*), 1.53 (s, 3H, -C-C*H₃*), 3.34 (dd, 1 H, ²J_{6*-6} = 12.5 Hz, ³J_{6*-5} = 5 Hz, H-6*), 3.50 (dd, 1 H, ²J_{6-6*} = 12.5Hz, ³J₆₋₅ = 7.5Hz, H-6), 3.87-3.93 (m, 1 H, H-5), 4.18 (dd, 1 H, ³J₄₋₃ = 2.5 Hz ³J₄₋₅ = 7.5 Hz, H-4), 4.31 (dd, 1 H, ³J₃₋₂ = 2.5 Hz, ³J₃₋₄ = 2.5Hz, H-3), 4.61 (dd, 1 H, ³J₂₋₁ = 7.5Hz, ³J₂₋₃ = 2.5Hz, H-2), 5.53 (d, 1 H, ³J₁₋₂ = 5Hz, H-1); ¹³C-NMR (250 MHz, CDCl₃) δ (ppm): 109.5, 108.7, 96.3, 71.0, 70.7, 70.3, 66.9, 50.6, 25.9, 25.9, 24.8, 24.3; ESI-MS (ion trap): m/z 286 [M+H]⁺.

**(1,2:3,4-di-*O*-isopropylidene-6-deoxy-amino)-D-galactopiranose (12; DIG-NH₂):** A solution of triphenylphosphine (5.49 g, 20.9 mmol, 1.3 eq.) in THF (35 mL) was added dropwise to a solution of **11** (4.60 g, 16.1 mmol, 1 eq.) in THF (60 mL), and the mixture was stirred at room temperature for 4 hours under nitrogen. Distilled water (25 mL) was than added and the resulting mixture was heated to reflux and vigorously stirred for 15 hours. Solvents were removed under vacuum and the residue was purified by flash chromatography using CH₂Cl₂:MeOH 95:5 + 1% of triethylamine as eluent to afford 4.05 g of **12** (97 % yield). ¹H-NMR (250 MHz, CDCl₃) δ (ppm): 1.32 (s, 6H, 2 ×-C-C*H₃*), 1.34 (s, 2H, -N*H₂*), 1.43 (s, 3H, -C-C*H₃*), 1.51 (s, 3H, -C-C*H₃*), 2.82 (dd, 1 H, ²J_{6*6} = 15 Hz, ³J_{6*-5} = 5 Hz, H-6*), 2.95 (dd, 1 H, ²J_{6-6*} = 12.5 Hz, ³J₆₋₅ = 7.5 Hz, H-6), 3.65-3.71 (m, 1 H, H-5), 4.21 (dd, 1H, ³J₄₋₃ = 7.5 Hz, ³J₄₋₅ = 2.5 Hz, H-4), 4.30 (dd, 1 H, ³J₃₋₂ = 5 Hz ³J₃₋₄ = 2.5Hz, H-3), 4.58 (dd, 1H, ³J₂₋₁ = 10 Hz, ³J₂₋₃ = 2.5 Hz, H-2), 5.53 (d, 1 H, ³J₁₋₂ = 2.5Hz, H-1); ¹³C-NMR (250 MHz, CDCl₃) δ (ppm): 109.1, 108.4, 96.3, 71.7, 70.7, 70.5, 69.4, 42.3, 26.0, 25.9, 24.9, 24.3; ESI-MS (ion trap): m/z 260 [M+H]⁺.

**N-[(1,2:3,4-di-*O*-isopropylidene-6-deoxy-D-galactopyranosyl)-carbamoyl]-p nitrophenol (13; DIG-PNPC):** A solution of **12** (1.0 g, 3.9 mmol, 1 eq.) in THF (15 mL) was added dropwise to a solution of bis (4-nitrophenyl) carbonate (1.17 g, 3.9 mmol, 1 eq.) and DMAP (1.01 g, 7.8 mmol, 2 eq.) in THF (20 mL) and the mixture was stirred at room temperature for 5 hours. The resulting mixture was diluted in EtOAc (150 mL) and washed with 0.5 N HCl (3 × 100 mL). The organic layer was dried over MgSO₄ and filtered. The solvent was evaporated under reduced pressure and the residue was purified by flash chromatography using CH₂Cl₂:Hexane 75:25 as eluent to afford 1.27 g of **13** (78 % yield). ¹H-NMR (250 MHz, CDCl₃) δ (ppm): 1.34 (s, 3H, -C-C*H₃*), 1.35 (s, 3H, -C-C*H₃*), 1.46 (s, 3H, -C-C*H₃*), 1.51 (s, 3H, -C-C*H₃*), 3.34-3.44 (m, 1 H, H-6*), 3.55-3.65 (m, 1 H, H-6), 3.95-4.01 (m, 1 H, H-5), 4.23 (dd, 1 H, ³J₄₋₃ = 10 Hz, ³J₄₋₅ = 2.5 Hz, H-4), 4.34 (dd, 1 H, ³J₃₋₂ = 2.5 Hz, ³J₃₋₄ = 5Hz, H-3), 4.63 (dd, 1 H, ³J₂₋₁ = 7.5Hz, ³J₂₋₃ = 2.5Hz, H-2), 5.55 (d, 1 H, ³J₁₋₂ = 5 Hz, H-1), 5.60 (dd, 1 H, ³J_{NH-6*} = 7.5 Hz, J_{NH-6} = 5Hz, - NH), 7.28-7.32 (m, 2H, H-2', H-6'), 8.21-8-25 (m, 2H, H-3', H-5'); ¹³C-NMR (250 MHz, CDCl₃) δ (ppm): 155.9, 153.3, 144.7, 125.0, 121.9, 109.5, 108.8, 96.3, 71.5, 70.7, 70.4, 66.0, 41.8, 26.0, 25.9, 24.9, 24.3; ESI-MS (ion trap): m/z 425 [M+H]⁺.

**3,4',5-tri-[N-(6-deoxy-galactosyl)-carbamoyl]-resveratrol (15; Resv(DGAL-C)₃):** A solution of resveratrol (0.15 g, 0.66 mmol, 1 eq.) and DMAP (0.32 g, 2.64 mmol, 4 eq.) in pyridine (5 mL) was added to a solution of **13** (1.40 g, 3.3 mmol, 5 eq) in pyridine (5 mL) and the resulting mixture was allowed to react under vigorous stirring at 45°C for 48 hours. The reaction mixture was then diluted in EtOAc (150 mL) and washed with 0.5 N HCl (7 × 100 mL). The organic layer was dried over MgSO₄ and filtered. The solvent was evaporated under reduced pressure and the residue was purified by flash chromatography using EtOAc:Hexane 5.5:4.5 as eluent to afford 0.55 g of **14** containing traces of the disubstituted resveratrol. **14** (0.5 g) was added, without further purification, to a solution of TFA/water (90/10, 5 mL). After 1.5 hours the resulting mixture was precipitated with 25 mL of diethyl ether, the material was centrifuged and the solvents were decanted. The white powder was then washed with diethyl ether three more times to eliminate traces of TFA. The precipitate was dissolved in water and, after lyophilization, purified using a reverse-phase preparative HPLC column (ACE 5AQ 150 mm × 21.2 mm from 0 % to 38 % of ACN in water in 17 minutes) to afford 0.31 g of **15** (79.7 % yield) as a white powder. ¹H-NMR (600 MHz, DMSO-d₆) δ (ppm): 2.98-4.95 (m, 33H, 3 × (H-1, H-2, H-3, H-4, H-5, H-6, H-6* galactose), 12 ×-OH), 6.78 (s, 1 H, H-4 resveratrol), 7.11-7.33 (m, 6H, H-3', H-5', H-2, H-6, H-8, H-9 resveratrol), 7.60 (d, 2H, ³J_{2'-3'} = 6Hz, H-2', H-6'), 7.68-7.81 (m, 3H, 3 × -N*H*); ¹³C-NMR (600 MHz, DMSO-d₆) δ (ppm): 154.4, 154.2, 151.7, 150.8, 138.9, 134.5, 133.5, 129.2, 127.5, 126.7, 122.0, 116.3, 101.7, 97.5, 92.7, 82.6, 82.1, 76.1, 73.3, 72.4, 71.9, 69.4, 69.2, 68.9, 68.6, 68.0, 68.0, 41.8, 41.7; ESI-MS (ion trap): m/z 845 [M+H]⁺.
**Solubility in water:** > 0.2 M
**Log P:** -0.36

### Example 4 Synthesis of 3,4',5-tri-[N-methyl-carbamoyl] resveratrol (Resv(Me-C)₃)

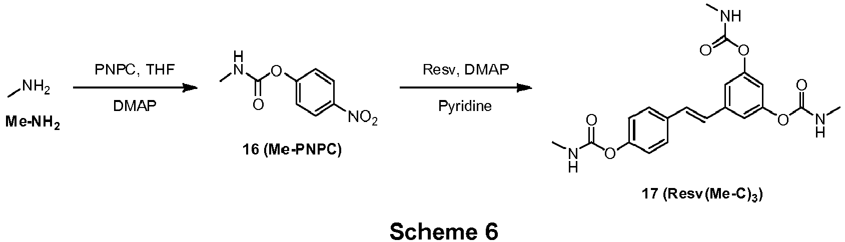

**[N-methyl-carbamoyl]-p-nitrophenol (16; Me-PNPC):** A solution of methyl amine (33% in ethanol, 1 mL, 0.25 g, 8.1 mmol, 1 eq.) in THF (10 mL) was added dropwise to a solution of bis (4-nitrophenyl) carbonate (2.45 g, 8.1 mmol, 1 eq.) and DMAP (1.0 g, 8.1 mmol, 1 eq.) in THF (10 mL), and the mixture was stirred at room temperature for 3 hours. The resulting mixture was diluted in CH₂Cl₂ (150 mL) and washed with 0.5 N HCl (3 × 100 mL). The organic layer was dried over MgSO₄ and filtered. The solvent was evaporated under reduced pressure and the residue was purified by flash chromatography using ethyl acetate:diethyl ether:hexane 3:2:5 as eluent to afford 1.2 g of **16** (76 % yield) as a pale yellow solid. ¹H-NMR (250 MHz, CDCl₃) δ (ppm): 2.92 (d, 3H, C*H₃*-NH-, ³J_{H-H} = 5.0 Hz), 5.06-5.21 (m, 1 H, CH₃-N*H*-), 7.30 (d, 2H, 2 × Ar-H, ³J_{H-H} = 8.75 Hz), 8.24 (d, 2H, 2 × Ar-*H*, ³J_{H-H} = 8.75 Hz); ¹³C-NMR (250 MHz, CDCl₃) δ (ppm): 155.9, 153.7, 144.7, 125.1, 121.9, 27.8; ESI-MS (ion trap): m/z 197 [M+H]⁺. **3,4',5-tri-[N-methyl-carbamoyl]-resveratrol (17; Resv(Me-C)₃):** A solution of resveratrol (0.27 g, 1.2 mmol, 1 eq.) and DMAP (0.72 g, 5.9 mmol, 5 eq.) in pyridine (5 mL) was added to a solution of **16** (1.16 g, 5.9 mmol, 5 eq) in pyridine (5 mL) and the resulting mixture was allowed to react under vigorous stirring at 50°C for 48 hours. The reaction mixture was then diluted in CH₂Cl₂ (150 mL) and washed with 0.5 N HCl (2 × 100 mL). The organic layer was dried over MgSO₄ and filtered. The solvent was evaporated under reduced pressure and the residue was purified by flash chromatography using acetone:hexane 5:5 as eluent to afford 0.35 g of **17** (73% yield) as a yellow solid. ¹H-NMR (250 MHz, CDCl₃) δ (ppm): 2.66-2.69 (m, 9H, 3 × C*H₃*-NH-), 6.78 (t, 1 H, ⁴J_{H-H} = 2 Hz, H-4), 7.10-7.42 (m, 6H, H-3', H-5', H-2, H-6, H-8, H-9), 7.58-7.71 (m, 5H, 3 × CH₃-N*H*-, H-2', H-6'); ¹³C-NMR (250 MHz, CDCl₃) δ (ppm): 154.7, 154.5, 151.7, 150.8, 138.8, 133.5, 129.1, 127.4, 126.6, 126.1, 122.0, 116.2, 115.8, 27.0; ESI-MS (ion trap): m/z 400 [M+H]⁺.
**Solubility in water:** below detection limit
**Log P value:** 2.75

### Example 5 Synthesis of 3,4',5-tri-[N-(2,3-dihydroxypropyl)-carbamoyl] resveratrol (Resv(MDHP-C)₃)

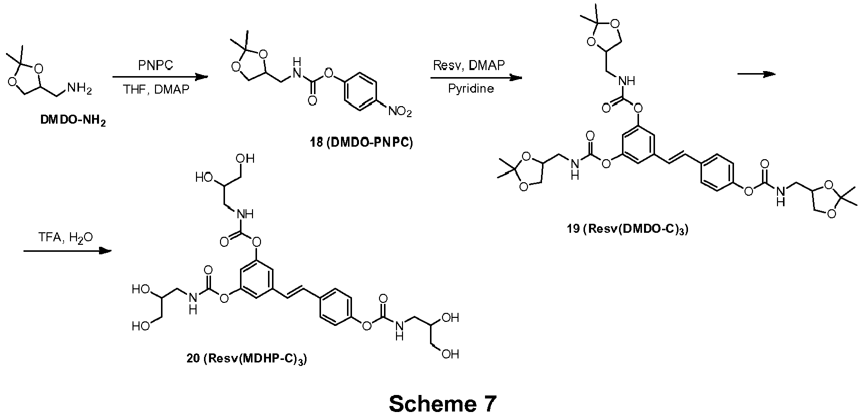

**[N-(2,2-dimethyl-1,3-dioxolane-4-methan)-carbamoyl]-p-nitrophenol (18; DMDO-PNPC):** 3.47 g (11.4 mmol, 1 eq.) of bis-p-nitrophenylcarbonate in 20 mL of anhydrous THF and 2.79 g (22.8 mmol, 2 eq.) of DMAP were mixed in a 100 mL round-bottom flask with a magnetic stirrer under N₂. 1.5 g (11.4 mmol, 1 eq.) of 2,2-dimethyl-1,3-dioxolane-4-methanamine in 20 mL of THF were then added dropwise at R.T. After three hours the mixture was taken up in EtOAc (150 mL), transferred to a 500 mL separation funnel and washed three times with 150 mL of 0.3 M HCl. The organic phase was then dried with MgSO₄ and filtered. The solvent was evaporated under reduced pressure and the desired product, **18,** was purified by flash chromatography on silica gel (eluent: 9.5 CH₂Cl₂/0.5 EtOAc). Yield: 75%. ¹H-NMR (250 MHz, CDCl₃) δ (ppm): 1.38 (s, 3H, -*CH₃*), 1.48 (s, 3H, -*CH₃*), 3.28-3.61 (m, 2H, -NH-C*H₂*), 3.70-4.14 (m, 2H, -C*H₂*-), 4.28-4.37 (m, 1 H, -C*H*-), 7.32 (d, 2H, Ar-*H*, ³J_{H-H}: 9.25 Hz), 8.25 (d, 2H, Ar-*H*, ³J_{H-H}: 9.25 Hz); ¹³C-NMR (62.9 MHz, CDCl₃) δ (ppm): 155.7, 153.3, 144.7, 125.1, 121.9, 109.6, 74.2, 66.5, 43.6, 26.7, 25.0; ESI-MS (ion trap): m/z 297 [M+H]⁺.

**3,4',5-tri-[N-(2,2-dimethyl-1,3-dioxolane-4-methan)-carbamoyl]-resveratrol (19; Resv(DMDO-C)₃):** 1.50 g (5.1 mmol, 6.0 equiv.) of **18** in 8 mL of pyridine were placed in a 100 mL round-bottom flask with magnetic stirrer and under N₂. 0.20 g (0.9 mmol, 1 equiv.) of resveratrol and e 0.43 g (3.5 mmol, 4 equiv.) of DMAP in 8 mL of pyridine were added and the reaction was allowed to proceed at 60°C overnight. The mixture was then taken up with 150 mL of EtOAc, transferred to a separation funnel and washed 5 times with 150 mL of 0.5 M HCl. The organic phase was then dried with MgSO₄ and filtered. The solvent was evaporated under reduced pressure. The resulting solid (0.42 g), which comprises traces of di-substitution products along with the tri-substituted compound, was purified by flash chromatography (eluent: 7.5 CH₂Cl₂/2.5 acetone) and used as such for the subsequent synthetic step.

**3,4',5-tri-[N-(2,3-dihydroxypropyl)-carbamoyl]-resveratrol (20; Resv(MDHP-C)₃):** 0.42 g of the mixture obtained in the previous step were placed in a 50 mL round-bottom flask, and 5 mL of a TFA/H₂O (9:1) solution were added. The resulting mixture was vigorously stirred for 1.5 h and then dried under vacuum. The product was separated by preparative HPLC (from 0% to 40% ACN in 17 minutes) and lyophilized to afford 0.33 g of **20** (cumulative yield of the final two steps: 63%). ¹H-NMR (250 MHz, CDCl₃) δ (ppm): ¹H-NMR (250 MHz DMSO-d₆) δ (ppm): 2.94-3.25 (m, 6H, 3 × -C*H₂*-), 3.35 (d, 6H, ³J_{H-H} = 5.25 Hz, 3 × -C*H₂*-), 3.53-3.62 (m, 3H, 3 × -C*H*-), 6.78 (t, 1 H, ⁴J_{H,H} = 2.0 Hz, H-4), 7.10-7.36 (m, 6H, H-2, H-6, H-3', H-5', H-7, H-8), 7.59-7.74 (m, 5H, H-2', H-6', 3 × -N-H); ¹³C-NMR (62.9 MHz, DMSO-d₆) δ (ppm): 154.4, 154.2, 151.7, 150.8, 138.8, 133.5, 129.1, 127.5, 126.7, 122.0, 116.2, 114.6, 70.3, 63.8, 44.1; ESI-MS (ion trap): m/z 580 [M+H]⁺.
**Solubility in water:** > 0.2 M
**Log P value:** -0.62

### Example 6 Synthesis of 3,4',5-tri-[N-(octadec-9-enyl)-carbamoyl]-resveratrol

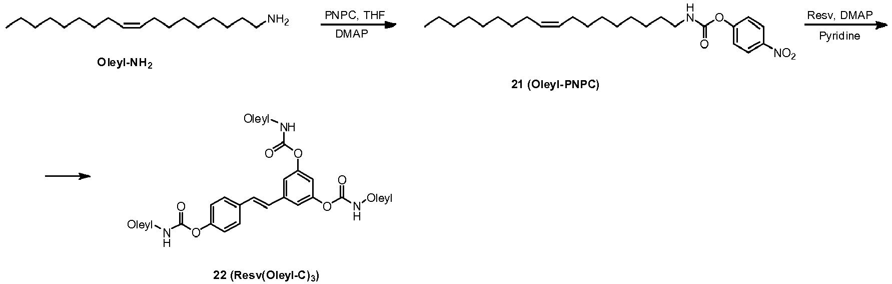

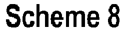

**[N-(octadec-9-enyl)-carbamoyl]-p-nitrophenol (21; Oleyl-PNPC):** A solution of octadec-9-enyl amine (2.67 g, 10.0 mmol, 1 eq.) in THF (10 mL) was added dropwise to a solution of bis (4-nitrophenyl) carbonate (3.32 g g, 10.9 mmol, 1.1 eq.) and DMAP (2.44 g, 19.9 mmol, 2 eq.) in THF (30 mL), and the mixture was stirred at room temperature for 3 hours. The resulting mixture was diluted in CH₂Cl₂ (150 mL) and washed with 0.5 N HCl (3 × 100 mL). The organic layer was dried over MgSO₄ and filtered. The solvent was evaporated under reduced pressure and the residue was purified by flash chromatography using acetone:hexane 2:8 as eluent to afford 4.1 g of **21** (94 % yield). ¹H-NMR (250 MHz, CDCl₃) δ (ppm): 1.17 (t, 3H, -C*H₃*, ³J_{H-H} = 6.75 Hz), 1.43-1.72 (m, 22H, 11 × -C*H₂*-), 1.84-1.90 (m, 2H, -C*H₂*), 2.27-2.32 (m, 4H, 2 × -C*H₂*-), 3.52-3.60 (m, 2H, -C*H₂*-), 5.52-5.66 (m, 3H, -N*H*-, -C*H*=C*H*-), 7.59 (d, 2H, 2 × Ar-*H*, ³J_{H-H} = 8.75 Hz), 8.51 (d, 2H, 2 × Ar-*H*, ³J_{H-H} = 8.75 Hz); ¹³C-NMR (250 MHz, CDCl₃) δ (ppm): 156.0, 153.0, 144.5, 129.9, 129.7, 125.0, 121.8, 41.3, 32.5, 31.8, 29.7, 29.6, 29.6, 29.5, 29.4, 29.2, 29.1, 27.1, 27.1, 26.6, 22.6, 14.0; ESI-MS (ion trap): m/z 433 [M+H]⁺.

**3,4',5-tri-[N-(octadec-9-enyl)-carbamoyl]-resveratrol (22; Resv(Oleyl-C)₃):** A solution of resveratrol (0.3 g, 1.3 mmol, 1 eq.) and DMAP (0.64 g, 5.2 mmol, 4 eq.) in pyridine (10 mL) was added to a solution of **21** (3.38 g, 7.8 mmol, 6 eq) in pyridine (5 mL) and the resulting mixture was allowed to react under vigorous stirring at 45°C for 48 hours. The reaction mixture was then diluted in CH₂Cl₂ (150 mL) and washed with 0.5 N HCl (7 × 100 mL). The organic layer was dried over MgSO₄ and filtered. The solvent was evaporated under reduced pressure and the residue was purified by flash chromatography using diethyl ether:hexane 3:7 as eluent until the exit of p-nitrophenol from the column, and hexane:EtOAc 7.5:2.5 thereafter, to afford 1.2 g of **22** (82% yield) as a white solid. ¹H-NMR (250 MHz, CDCl₃) δ (ppm): 0.85-91 (m, 9H, 3 × -*CH₃*), 1.16-1.56 (m, 72H, 36 × -C*H₂*-), 1.90-2.14 (m, 12H, 6 × -C*H₂*-), 3.19-3.28 (m, 6H, 3 ×-C*H₂*-), 5.10-5.48 (m, 9H, 3 × -N*H*-, 3 × -C*H*=C*H*-), 6.84-7.12 (m, 7H, H-4, H-3', H-5', H-2, H-6, H-8, H-9 resveratrol), 7.42 (d, 2H, ³J_{2'-3'} = 8.25 Hz, H-2', H-6'); ¹³C-NMR (250 MHz, CDCl₃) δ (ppm): 154.4, 154.1, 151.6, 150.7, 139.1, 133.9, 129.9, 129.7, 129.2, 127.4, 127.1, 121.7, 116.2, 114.3, 41.3, 32.6, 31.9, 31.7, 30.2, 29.7, 29.7, 29.7, 29.6, 29.6, 29.5, 29.5, 29.4, 29.4, 29.3, 29.3, 29.2, 29.2, 29.2, 28.9, 27.2, 26.9, 26.7, 22.6, 14.1; ESI-MS (ion trap): m/z 1109 [M+H]⁺.
**Solubility in water:** below detection limit
**Log P value:** 10.7

### Example 7 Synthesis of 3,4',5-tri-[N-(glycyl)-carbamoyl]-resveratrol:

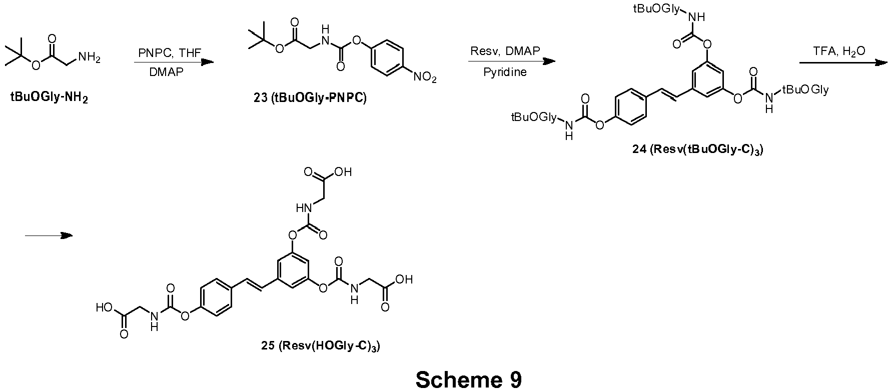

**[N-(tert-butyl-glycyl)-carbamoyl]-p-nitrophenol (23; tBuOGly-PNPC):** A solution of glycine tert-butyl ester hydrochloride (1.0 g, 4.0 mmol, 1 eq.) and DMAP (0.7 g, 4.0 mmol, 1 eq.) in CH₂Cl₂ (10 mL) was added dropwise to a solution of bis (4-nitrophenyl) carbonate (1.8 g, 4.0 mmol, 1 eq.) and DMAP (0.7 g, 4.0 mmol, 1 eq.) in THF (20 mL), and the mixture was stirred at room temperature for 3 hours. The resulting mixture was diluted in CH₂Cl₂ (150 mL) and washed with 0.5 N HCl (3 × 100 mL). The organic layer was dried over MgSO₄ and filtered. The solvent was evaporated under reduced pressure and the residue was purified by flash chromatography using chloroform:acetone:hexane 3:1:6 as eluent to afford 1.26 g of **23** (72 % yield). ¹H-NMR (250 MHz, CDCl₃) δ (ppm): 1.49 (s, 9H, 3 × -C-C*H₃*), 3.95 (d, 2H, -C*H₂*-, ³J_{H-H} = 5.25 Hz), 5.71 (t, 1 H, -N*H*-, ³J_{H-H} = 5.25 Hz), 7.31 (d, 2H, 2 × Ar-*H*, ³J_{H-H} = 9.25 Hz), 8.23 (d, 2H, 2 × Ar-*H*, ³J_{H-H} = 9.25 Hz); ¹³C-NMR (250 MHz, CDCl₃) δ (ppm): 168.4, 155.7, 153.0, 144.8, 125.1, 122.0, 82.8, 43.4, 28.0; ESI-MS (ion trap): m/z 297 [M+H]⁺.

**3,4',5-tri-[N-(tert-butyl-glycyl)-carbamoyl]-resveratrol (24; Resv(tBuOGly-C)₃):** A solution of resveratrol (0.16 g, 0.7 mmol, 1 eq.) and DMAP (0.35 g, 2.8 mmol, 4 eq.) in pyridine (10 mL) was added to a solution of **23** (1.26 g, 4.3 mmol, 6 eq) in pyridine (5 mL) and the resulting mixture was allowed to react under vigorous stirring at 45 °C for 48 hours. The reaction mixture was then diluted in CH₂Cl₂ (150 mL) and washed with 0.5 N HCl (7 × 100 mL). The organic layer was dried over MgSO₄ and filtered. The solvent was evaporated under reduced pressure and the residue was purified by flash chromatography using dichloromethane:diethyl ether:hexane 6:2:2 as eluent to afford 0.42 g of **24** (83 % yield). ¹H-NMR (250 MHz, CDCl₃) δ (ppm): 1.47 (s, 27H, 9 × -C-C*H₃*), 3.91-3.93 (m, 6H, 3 × -C*H₂*-), 5.77-5.84 (m, 3H, 3 × -N*H*-), 5.10-5.48 (m, 9H, 3 × -N*H*-, 3 × -C*H*=C*H*-), 6.80-7.09 (m, 7H, H-4, H-3', H-5', H-2, H-6, H-8, H-9), 7.38 (d, 2H, ³J_{2'-3'} = 8.5 Hz, H-2', H-6'); ¹³C-NMR (250 MHz, CDCl₃) δ (ppm): 168.8, 168.7, 154.4, 154.1, 151.3, 150.4, 139.1, 134.0, 129.2, 127.4, 126.9, 121.7, 116.4, 82.3, 43.3, 27.9; ESI-MS (ion trap): m/z 700 [M+H]⁺.

**3,4',5-tri-[N-(glycyl)-carbamoyl]-resveratrol (25; Resv(HOGly-C)₃): 24** (0.42 g, 0.6 mmol) was dissolved in a solution of TFA/water (4 mL, 97.5/2.5). After stirring for 1 h at room temperature, the solvents were evaporated under reduced pressure and the residue was dissolved in water/DMSO 1:1 and purified using a reverse-phase preparative HPLC column (ACE 5AQ 150 mm × 21.2 mm from 10 % to 40 % of ACN in water in 13 minutes) to afford 0.3 g of **25** (94 % yield) as a white powder after freeze drying. ¹H-NMR (250 MHz, DMSO-d₆) δ (ppm): 3.70-3.86 (m, 6H, 3 × -C*H₂*-), 5.10-5.48 (m, 9H, 3 × -N*H*-, 3 × -C*H*=C*H*-), 6.79 (t, 1 H, ⁴J₄₋₂ = 2 Hz, H-4), 7.11-7.38 (m, 6H, H-3', H-5', H-2, H-6, H-8, H-9), 7.63 (d, 2H, ³J_{2'-3'} = 8.75 Hz, H-2', H-6'), 8.09-8.20 (m, 3H, 3 × -N*H*-); ¹³C-NMR (250 MHz, DMSO-d₆) δ (ppm): 171.2, 171.2, 154.7, 154.5, 151.6, 150.6, 139.1, 133.6, 129.3, 127.6, 126.7, 121.9, 118.5, 116.2, 114.3, 42.2; ESI-MS (ion trap): m/z 532 [M+H]⁺.
**Solubility in water:** > 2*10⁻² M
**Log P:** 1.58

### Example 8 Synthesis of 3,4',5-tri-[N-(phenylalanyl)-carbamoyl]-resveratrol:

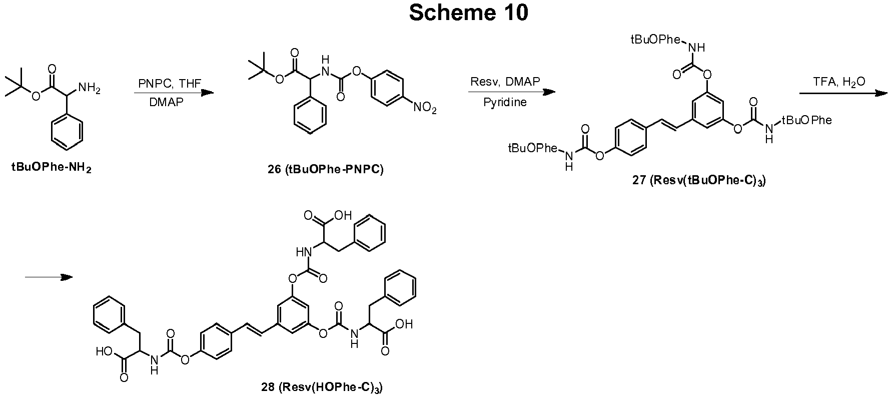

**[N-(tert-butyl-phenylalanyl)-carbamoyl]-p-nitrophenol (26; tBuOPhe-PNPC):** A solution of phenylalanine tert-butyl ester hydrochloride (2.36 g, 9.15 mmol, 1 eq) and DMAP (1.12 g, 9.15 mmol, 1 eq) was added dropwise to a solution of bis (4-nitrophenyl) carbonate (2.78 g, 9.15 mmol, 1 eq) and DMAP (1.12 g, 9.15 mmol, 1 eq), and the mixture was stirred at room temperature for 4 hours. The resulting mixture was diluted in CH₂Cl₂ (200 mL) and washed with 0.5 N HCl (3 × 150 mL). The organic layer was dried over MgSO₄ and filtered. The solvent was evaporated under reduced pressure and the residue was purified by flash chromatography using CH₂Cl₂:acetone = 9:1 as eluent, to afford 2.86 g of **26** (81 % yield). 1 H-NMR (250 MHz, CDCl3) δ (ppm): 1.54 (s, 9H, 3 × -C-CH3), 3.05-3.35 (m, 2H, 2 × C-CH2), 4.70 (dd, 1 H, -NH-CH-CH2- J = 14.7, 6.5 Hz), 6.33 (d, 1 H, -NH-CH-, J = 8.3 Hz), 7.22-7.45 (m, 7H, aromatic-H), 8.23 (d, 2 H, PNP aromatic-H, J= 15.5 Hz); 13C-NMR (250 MHz, CDCl₃) δ (ppm): 170.4, 155.8, 152.6, 144.6, 136.0, 129.5, 128.1, 127.5, 124.9, 121.9, 82.7, 55.6, 38.2, 27.3.

**3,4',5-tri-[N-(tert-butyl-phenylalanyl)-carbamoyl]-resveratrol (27; Resv(tBuOPhe-C)₃):** A solution of resveratrol (0.24 g, 1.1 mmol, 1 eq.) and DMAP (0.52 g, 4.2 mmol, 4 eq.) in pyridine (10 mL) was added to a solution of **26** (1.84 g, 4.8 mmol, 4.5 eq) in pyridine (5 mL) and the resulting mixture was allowed to react under vigorous stirring at 40 °C for 48 h. The reaction mixture was diluted in ethyl acetate (200 mL) and washed with 0.5 N HCl (3 × 250 mL). The organic layer was dried over MgSO₄ and filtered. The solvent was evaporated under reduced pressure and the residue was purified by flash chromatography using hexane:diethyl ether=7:3 as solvent until the p-nitrophenol was totally eluted, and then using hexane:diethyl ether:ethyl acetate=5:3.5:1.5 as solvent to afford 0.590 g of **27** (57 % yield). ¹H-NMR (250 MHz, CDCl₃) δ (ppm): 1.41 (s, 27H, 3 × C-C*H₃*), 3.02-3.30 (m, 6H, 3 × Ph-C*H₂*), 4.16 (q, 1 H, NH-*CH*-CH₂, J = 7.1 Hz), 4.64 (q, 2H, 2 × NH-C*H*-CH₂, J= 6.2 Hz), 5.79 (m, 3H, 3 ×-NH-), 6.81-7.56 (m, 24 H, aromatic-H); ¹³C-NMR (250 MHz, CDCl₃) δ (ppm): 170.6, 154.0, 153.7, 151.6, 150.8, 139.4, 136.2, 128.8, 128.7, 127.7, 127.3, 122.0, 116.7, 114.5, 82.8, 55.6, 38.6, 28.2.

**3,4',5-tri-[N-(phenylalanyl)-carbamoyl]-resveratrol (28; Resv(HOPhe-C)₃): 27** (0.59 g, 0.61 mmol) was dissolved in a solution of TFA (3.9 mL) and water (0.1 mL) After stirring for 1 h at room temperature, the solvents were evaporated under reduced pressure and the residue was dissolved in water/DMSO 1:1 and purified using a reverse-phase preparative HPLC column (ACE 5AQ 150 mm x 21.2 mm from 35 % to 65 % of ACN in water in 13 minutes) to afford 0.36 g of **28** (74 % yield). 1 H-NMR (250 MHz, CDCl3) δ (ppm): 3.59-4.04 (m, 6H, 3 × Ph-CH2), 4.96-5.11 (m, 3H, 3 × NH-CH-CH2), 7.70-8.45 (m, 24H, aromatic-H), 8.96-9.11 (m, 3H, 3 × -NH-); 13C-NMR (250 MHz, CDCl3) δ (ppm): 164.0, 145.1, 142.6, 141.7, 130.1, 128.9, 128.9, 124.7, 120.3, 119.4, 118.9, 117.7, 112.9, 107.2, 45.9, 30.6.
**Solubility in water:** below detection limit
**Log P:** 6.03

### Examples of hydrolysis studies

Assays were carried out as detailed in Examples, Methods.

Carbamate derivatives are stable at pH 1. They hydrolyse very slowly in mildly acidic media, as shown below (see point c). At near-neutral pH and in blood the kinetics of hydrolysis are similar for the various compounds. Two examples are shown (points a and b below).

### a. Kinetics of hydrolysis of 3,4',5-tri-(N-(6-deoxy-galactosyl)-carbamoyl)-resveratrol derivatives (15; Resv(DGAL-C)₃)

HPLC analysis of the samples was carried out using an RRLC-reverse phase column (Zorbax Eclipse Plus C18, 1.8 µm, 2.1 x 50 mm i.d.; Agilent), with the eluent composition and gradient as specified in Table 1.

**Table 1. Typical eluent composition and gradient used for monitoring hydrolysis of Resv(DGAL-C)₃.**

| **Time (min)** | **% A (H₂O + 0,1% TFA)** | **% B (ACN)** |
|---|---|---|
| 0 | 98 | 2 |
| 0,5 | 98 | 2 |
| 0,8 | 90 | 10 |
| 2,5 | 82 | 18 |
| 3 | 72 | 28 |
| 3,5 | 72 | 28 |
| 5,3 | 0 | 100 |
| 6,2 | 0 | 100 |
| 7 | 98 | 2 |
| 7,5 | 98 | 2 |

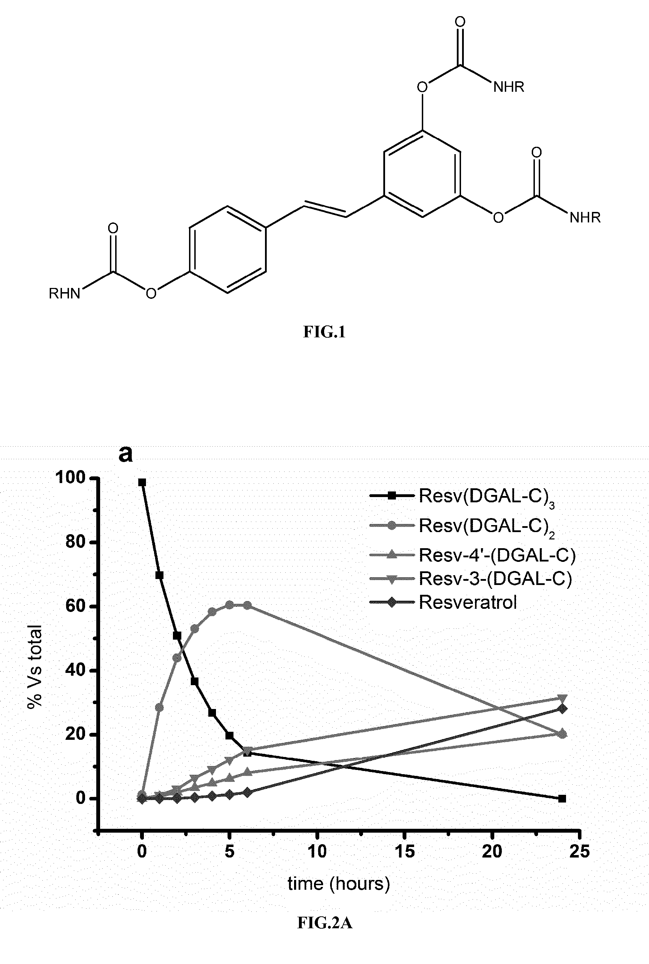

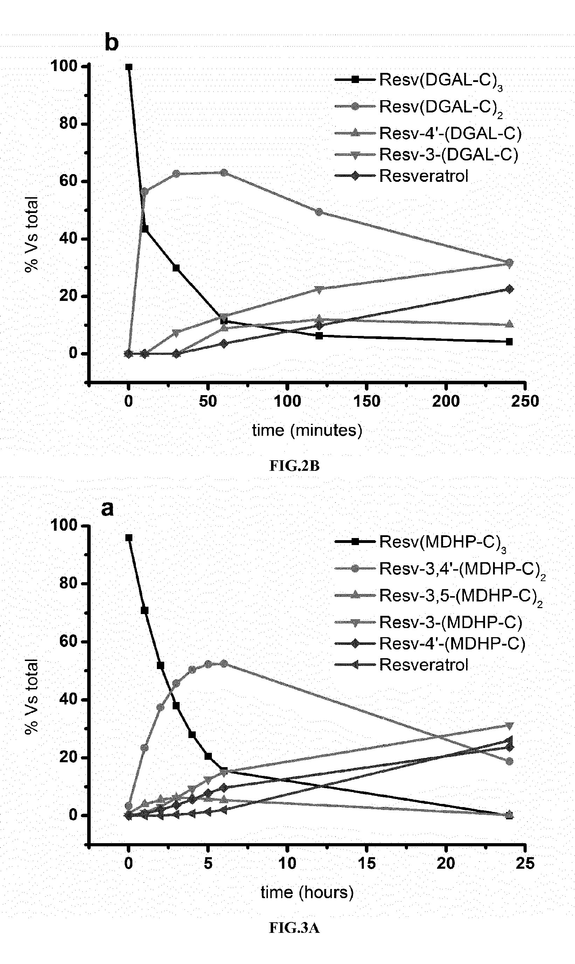

Representative time profiles for the concentration of Resv(DGAL-C)₃ and those of its hydrolysis products are shown in Fig. 2A) and Fig. 2B) for experiments conducted in Phosphate Buffered Saline (PBS) at pH 6.8 and in fresh rat blood, respectively.

### b. Kinetics of hydrolysis of (N-(2,3-hydroxypropyl)-carbamoyl)-resveratrol derivatives (20; Resv(MDHP-C)₃)

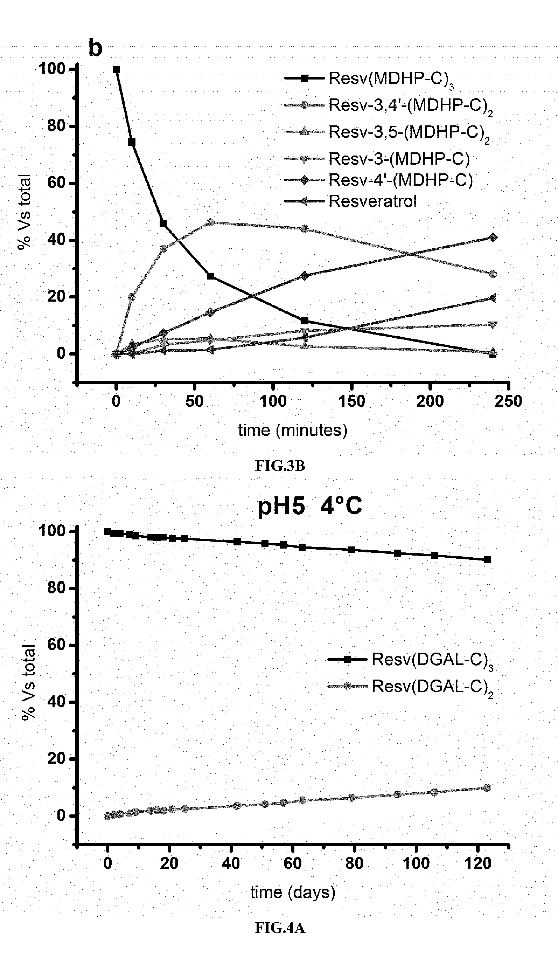

HPLC analysis of the samples was carried out as described in the previous example. Representative time profiles for the concentration of Resv(MDHP-C)₃ and those of its hydrolysis products are shown in Fig. 3A) and Fig. 3B) for experiments conducted in PBS at pH 6.8 and in fresh rat blood, respectively.

### c. Kinetics of hydrolysis of 3,4',5-tri-(N-(6-deoxy-galactosyl)-carbamoyl)-resveratrol derivatives (15; Resv(DGAL-C)₃) at pH 5 and at different temperatures

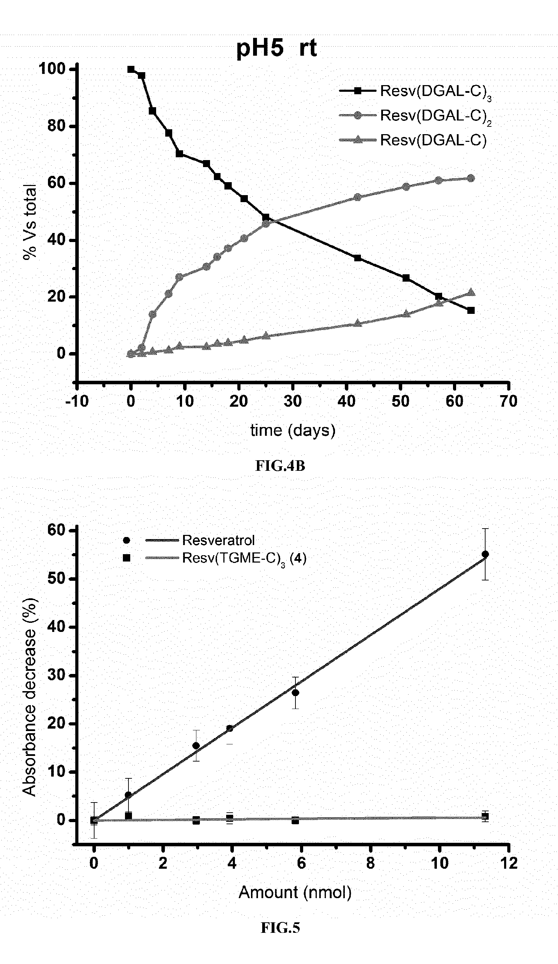

Representative time profiles for the concentration of Resv(DGAL-C)3 and those of its hydrolysis products are shown in Fig. 4A) and Fig. 4B) for experiments conducted in PBS at pH 5.0 at 4 °C and at room temperature, respectively.

### Protection against oxidative reactions

A representative result is reported in Fig. 5, showing that a carbamoyl-protected resveratrol derivative bearing methyl-capped trimeric-ethyleneglycol chains as the substituent groups, (Resv(TGME-C)3), does not react at all with DPPH, which causes instead stoichiometric oxidation of resveratrol.

The higher stability and the better resistance to oxidative degradation of the compounds according to the invention has been tested in addition to the compounds of Examples 1 to 8 also with the compounds indicated in the following list
- 3,4',5-tri-(N-leucyl-carbamoyl)-resveratrol
- 3,4',5-tri-(N-isoleucyl-carbamoyl)-resveratrol
- 3,4',5-tri-(N-threonyl-carbamoyl)-resveratrol
- 3,4',5-tri-[N-(methoxy tetra-(ethylenglycol))-carbamoyl]-resveratrol

All compounds gave significant results: all are stable under oxidizing conditions as assayed by the DPPH test; solubilities in water ranged between < 1 µM and > 50 mM depending on the nature of the substituent, providing the desired versatility for multiple applications. These results confirm that the advantageous properties of the compounds according to the invention are attributable to a wide class of resveratrol derivatives.

### REFERENCES

Agarwal B, Baur JA (2011) Resveratrol and life extension. Ann NY Acad Sci 1215:138-143.
Biasutto L, Marotta E, Garbisa S, Zoratti M, Paradisi C (2010) Determination of quercetin and resveratrol in whole blood - implications for bioavailability studies. Molecules 15:6570-6579.
Fukumoto LR, Mazza G (2000) Assessing antioxidant and prooxidant activities of phenolic compounds. J Agric Food Chem 48:3597-3604.
Harikumar KB, Aggarwal BB (2008) Resveratrol: a multitargeted agent for age-associated chronic diseases. Cell Cycle 7:1020-1035.
Lu Y, Yu B, Dong H, Hong T (2011) Resveratrol derivatives as antitumor agents and their preparation, pharmaceutical compositions and use in the treatment of cancer. Chinese patent CN 102126993, granted July 20, 2011
Maes D, Mohammadi F, Qu L, Czarnota A, Wammone T, Declercq L, Zecchino JR (2009) Emulsion cosmetic compositions containing resveratrol derivatives and an oil phase structuring agent. U.S. Patent Application 20090035236, published 02/05/2009.
Park SJ, Ahmad F, Philp A, Baar K, Williams T, Luo H, Ke H, Rehmann H, Taussig R, Brown AL, Kim MK, Beaven MA, Burgin AB, Manganiello V, Chung JH (2012) Resveratrol ameliorates aging-related metabolic phenotypes by inhibiting cAMP phosphodiesterases. Cell 148:421-433.
Pervaiz S, Holme AL (2009) Resveratrol: its biologic targets and functional activity. Antiox Redox Signal 11:2851-2897.
Pettit GR, Grealish MP (2010) Structural modification of resveratrol: Sodium resverastatin phosphate. US Patent Application 20050240062, filed on April 10, 2003. US Patent N. 7705188, issued Apr. 27, 2010. WO 01/60774.
Petrovski G, Gurusamy N, Das DK (2011) Resveratrol in cardiovascular health and disease. Ann NY Acad Sci 1215:22-33.
Soby L, Theophilus EH, Ptchelintsev D, Attar PS (2003) Resveratrol analogues. US Patent application US 2003/0118617 A1, published June 26, 2003.
Tetko IV, Bruneau P (2004) Application of ALOGPS to predict 1-octanol/water distribution coefficients, IgP, and logD, of Astra-Zeneca in-house database. J Pharm Sci. 93:3103-3110.

## Claims

1. A resveratrol derivative having the formula (I): 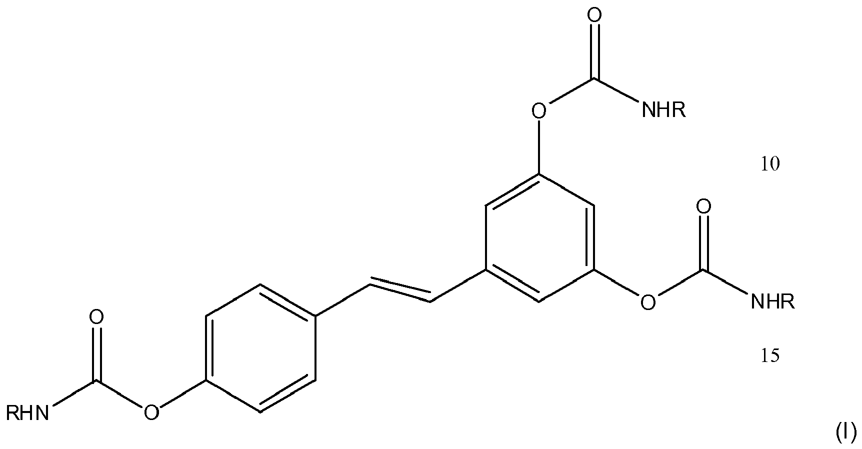 wherein each group NHR derives from the corresponding amine RNH₂ selected from the following compounds:
- a methoxyoligoethylene glycol amines selected from the group consisting of: methoxymonoethylene glycol amine, methoxydiethylene glycol amine, methoxytriethylene glycol amine, methoxytetraethylene glycol amine; methoxypentaethylene glycol amine, methoxyhexaethylene glycol amine, methoxyheptaethylene glycol amine, methoxyoctaethylene glycol amine, methoxynonaethylene glycol amine, methoxydecaethylene glycol amine, methoxypolyethylene glycol amine with an average Mₙ = 2,000, methoxypolyethylene glycol amine with an average Mₙ = 5,000, methoxypolyethylene glycol amine with an average Mₙ = 10,000, methoxypolyethylene glycol amine with an average Mₙ = 20,000.
- an amino deoxy sugars selected from the group consisting of: amino deoxy pentoses, amino deoxy hexoses, amino deoxy riboses, amino deoxy arabinoses, amino deoxy xyloses, amino deoxy lyxoses, amino deoxy alloses, amino deoxy altroses, amino deoxy glucoses, amino deoxy guloses, amino deoxy mannoses, amino deoxy galactoses, amino deoxy rhamnoses.
- an aliphatic amino alcohol or an aliphatic amino polyalcohol selected from the group consisting of: aminomethanol, aminoethanol, aminoethandiol, aminopropanol, aminopropandiol, aminopropantriol, aminobutanol, aminobutandiol, aminobutantriol, aminobutantetraol, aminopentanol, aminopentandiol, aminopentantriol, aminopentantetraol, aminopentanpentaol, aminocyclopentanol, aminocyclopentandiol, aminocyclopentantriol, aminocyclopentantetraol, aminocyclopentanpentaol, aminohexanol, aminohexandiol, aminohexantriol, aminohexantetraol, aminohexanpentanol, aminohexanhexaol, aminocyclohexanol, aminocyclohexandiol, aminocyclohexantriol, aminocyclohexantetraol, aminocyclohexanpentaol, aminocyclohexanhexaol aminoheptanol, aminoheptandiol, aminoheptanetriol, aminoheptanetetraol, aminoheptanpentaol, aminoheptanhexaol, aminoheptanheptaol, aminooctanol, aminooctandiol, aminooctantriol, aminooctanetetraol, aminooctanpentaol, aminooctanhexaol, aminooctaneheptaol, aminooctaneoctaol, aminononanol, aminononandiol, aminononantriol, aminononantetraol, aminononanpentaol, aminononanhexaol, aminononanheptaol, aminononanoctaol, aminononannonaol, aminodecanol, aminodecandiol, aminodecantriol, aminodecantetraol, aminodecanpentaol, aminodecanhexaol, aminodecanheptaol, aminodecanoctaol, aminodecannonanol, aminodecandecanol, and their aliphatic amino alcohols or aliphatic amino polyalcohols isomers.
- a natural or unnatural amino acid selected from the group consisting of: alanine, arginine, asparagine, cysteine, aspartic acid, glutamic acid, glutamine, glycine, ornithine, proline, selenocysteine, serine, taurine, tyrosine, histidine, isoleucine, leucine, lysine, methionine, phenylalanine, threonine, tryptophan, valine, 2,4-diaminobutyric acid, 2-aminoheptanoic acid, 2-aminohexadecanoic acid, 5-aminovaleric acid, 4-hydroxy-proline, 3-phenylserine, α-methylvaline, 2-aminocaprylic acid, 2-amino-2-phenylbutyric acid, 2,6-diaminopimelic acid, and their D or L stereoisomers.

2. The resveratrol derivative according to claim 1 selected from the group comprising the following compounds:
- 3,4',5-tri-[N-(methoxy tri-(ethylenglycol))-carbamoyl] resveratrol;
- 3,4',5-tri-[N-(methoxy tetra-(ethylenglycol))-carbamoyl] resveratrol;
- 3,4',5-tri-[N-(methoxy hexa-(ethylenglycol))-carbamoyl] resveratrol;
- 3,4',5-tri-[N-(6-deoxy-galactosyl)-carbamoyl]-resveratrol;
- 3,4',5-tri-[N-(2,3-dihydroxypropyl)-carbamoyl] resveratrol;
- 3,4',5-tri-[N-methyl-carbamoyl] resveratrol;
- 3,4',5-tri-[N-(octadec-9-enyl)-carbamoyl]-resveratrol;
- 3,4',5-tri-[N-(glycyl)-carbamoyl]-resveratrol;
- 3,4',5-tri-[N-(leucyl)-carbamoyl]-resveratrol;
- 3,4',5-tri-[N-(isoleucyl)-carbamoyl]-resveratrol;
- 3,4',5-tri-[N-(threonyl)-carbamoyl]-resveratrol;
- 3,4',5-tri-[N-(phenylalanyl)-carbamoyl]-resveratrol.

3. The resveratrol derivative according to claim 1 or 2 as medicament.

4. The resveratrol derivative according to the previous claim for use in the treatment of inflammatory conditions, viral infections, bacterial infections of the upper respiratory tracts, metabolic disease, cancer, disorders of the gastro-intestinal tract, cardiovascular disease, traumatic brain injury, disorders of skeletal muscle, of the central and peripheral nervous systems, of the endocrine, dermatological and genitourinary systems.

5. A pharmaceutical composition comprising the compound according to any one of the claims from 1 to 4 or acceptable salt thereof, and a pharmaceutically acceptable carrier.

6. The pharmaceutical composition according to claim 5 in the form of tablets, capsules, powders, granules, lozenges, creams, syrups, sprays, solutions or suspensions.

7. The pharmaceutical composition according to claim 5 or 6 for oral, topical or parenteral administration.

8. A cosmetic composition comprising the compound according to any one of the claims from 1 to 4 and a suitable carrier for topic administration.

9. A composition comprising the compound according to any one of the claims from 1 to 4 in the form of food supplement, food, nutraceutical or medical device, and one or more excipients and/or food ingredients.

10. A process for synthesizing the compounds of any one of the claims from 1 to 4 according to the following scheme, wherein R is as defined in the preceding claims and the obtained compound is isolated and optionally purified.

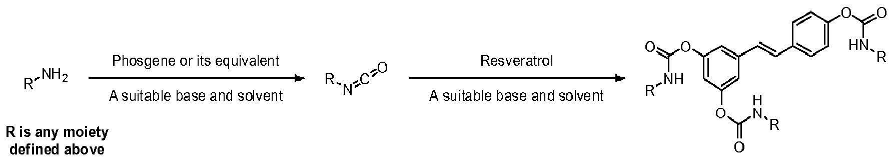

11. A process for synthesizing the compounds of any one of the claims from 1 to 4 according to the following scheme, wherein R is as defined in the preceding claims and the obtained compound is isolated and optionally purified.

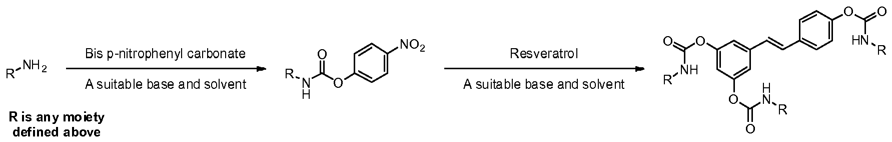

12. The process according to claim 10 or 11 wherein said suitable solvent is selected from: dichloromethane (CH₂Cl₂), chloroform (CHCl₃), N,N-dimethylformamide (DMF), tetrahydrofuran (THF), acetonitrile (ACN), ethyl acetate (EtOAc).

13. The process according to any one of the claims 10 or 11 wherein said suitable base is selected from: triethylamine, diisopropylethylamine, 4-(dimethylamino)pyridine, pyridine, sodium hydride.
